(19) Europäisches Patentamt
European Patent Office
Office européen des brevets

(11) Publication number: 0 257 275 A2

(12) # EUROPEAN PATENT APPLICATION

(21) Application number: 87110148.1

(22) Date of filing: 14.07.87

(51) Int. Cl.[4]: C 07 D 501/18, C 07 D 499/78

(30) Priority: 28.07.86 US 890000
20.10.86 US 920398
20.10.86 US 920397
20.10.86 US 920399

(43) Date of publication of application: 02.03.88 Bulletin 88/9

(84) Designated Contracting States: AT BE CH DE ES FR GB GR IT LI NL SE

(71) Applicant: AMERICAN CYANAMID COMPANY,
1937 West Main Street P.O. Box 60, Stamford Connecticut 06904-0060 (US)

(72) Inventor: Curran, William Vincent, 27 Harding Street, Pearl River New York 10965 (US)
Inventor: Babine, Robert, 2 Charles Lane, Apt. 2B, Pomona New York 10970 (US)
Inventor: Lee, Ving Jick, 19 Shuart Road, Monsey New York 10952 (US)

(74) Representative: Wächtershäuser, Günter, Dr., Tal 29, D-8000 München 2 (DE)

(54) **Cephalosporin and penicillin derivatives.**

(57) Cephalosporins and derivatives thereof, and processes for their preparation.

EP 0 257 275 A2

Title: Cephalosporins and Derivatives

This invention relates to compounds of the following formulae:

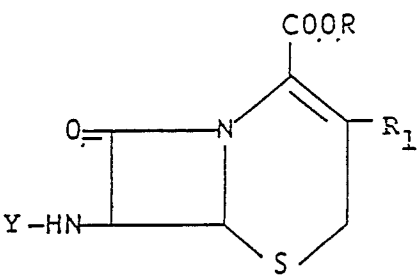

or

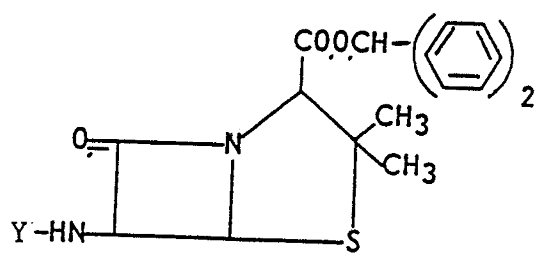

wherein R is hydrogen or diphenylmethyl; $R_1$ is hydrogen, alkyl($C_1$-$C_3$), vinyl, acetyloxymethyl, diphenylmethyl, $CH_2OCH_2CH_2Si(CH)_3$, $Si(CH_3)_3$ or

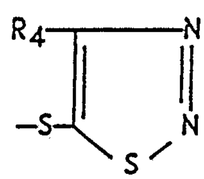

,

wherein $R_4$ is hydrogen or alkyl ($C_1$-$C_6$); and Y is

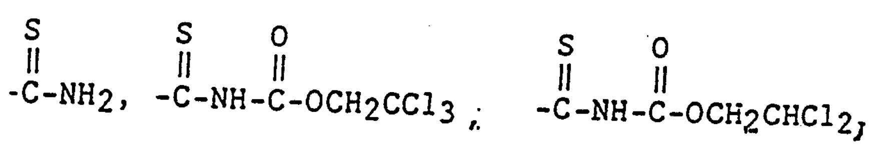

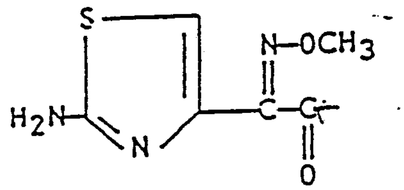

or

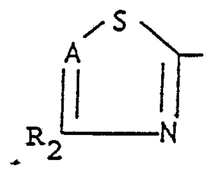

wherein A is HC, N or $R_3$-C, wherein $R_3$ is acetyl or benzyl; and $R_2$ is alkyl ($C_1$-$C_3$), phenyl, carboxylic acid, (2,2,2-trichloroethoxy)carbonyl, [2-(trimethylsilyl)ethoxy]-carbonyl, phenylmethylamino carbonyl or ethoxycarbonyl.

According to the present invention, novel intermediates are generated pursuant to reactions summarized in Scheme I.

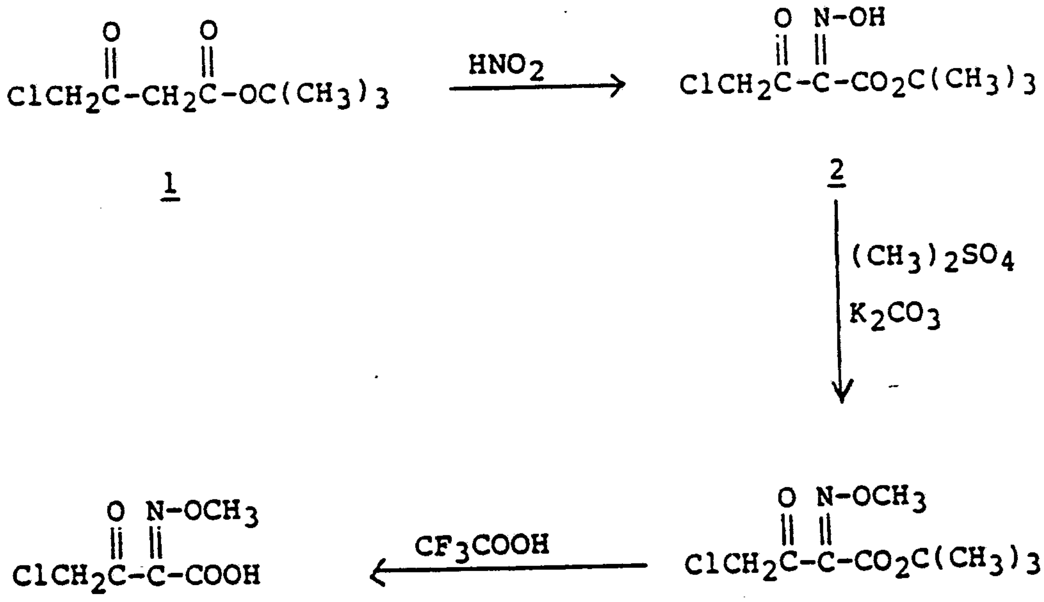


1 2 3 4

Nitrosation of the t-butyl ester 1 gave the oximino derivative 2 as an oil which was methylated with dimethyl sulfate and potassium carbonate in acetone to afford the methoxime 3. Compound 3 was purified by chromatography to

give the pure (Z)-methoximo compound as an oil which crystallized on standing.

Treatment of compound 3 with trifluoroacetic acid produced the desired (Z)-methoximo acid 4 as a white crystalline compound. Compounds 2, 3 and 4 are new compounds.

Scheme II

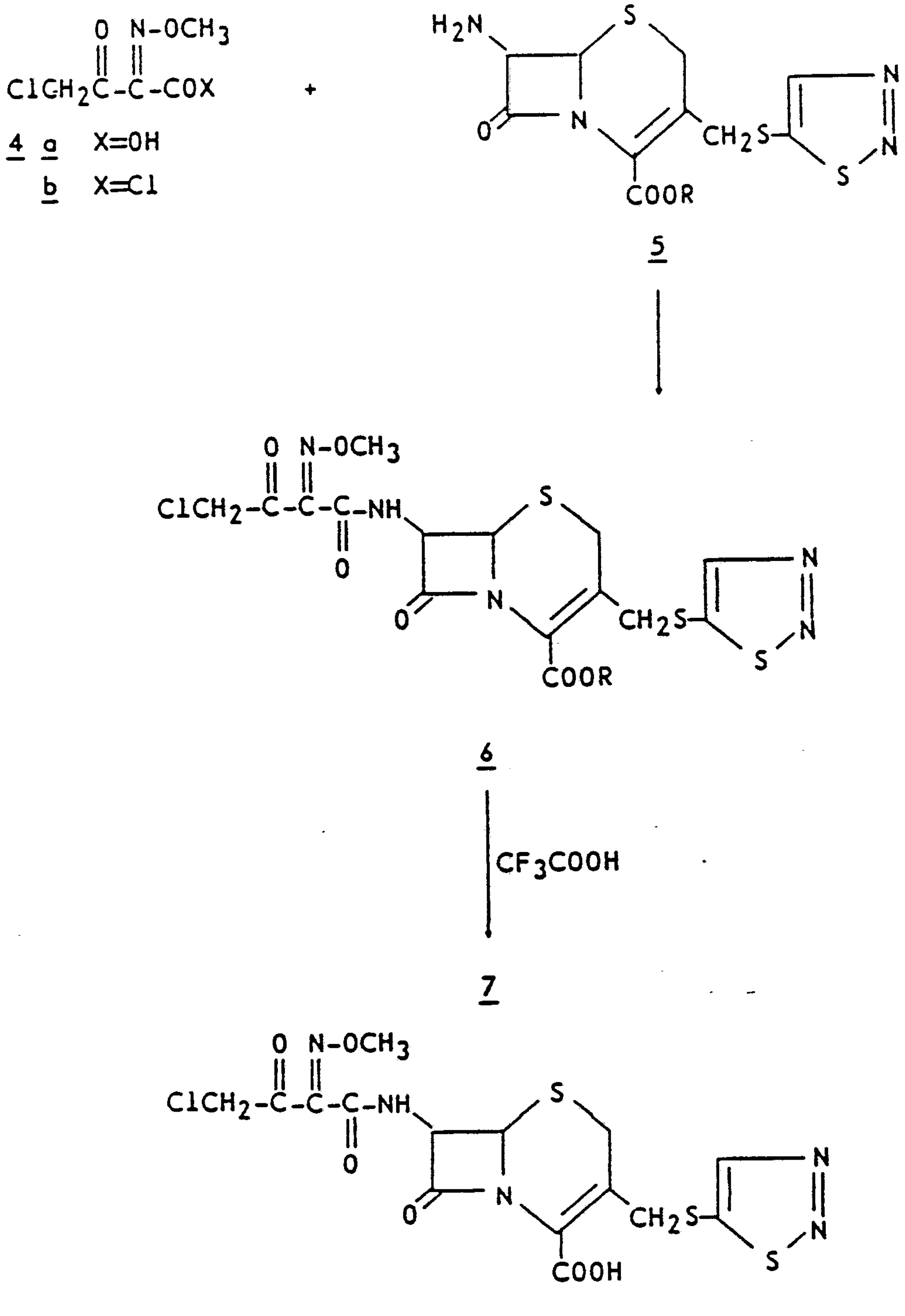


4 a X=OH

b X=Cl

5

6

7

7

a). R = $CH_2OCH_2CH_2Si(CH_3)_3$

b). R = $CH(C_6H_5)_2$

c). R = $Si(CH_3)_3$

The condensation of the chlorooximino acid 4 with the 7-aminocephalosporin compound, for examle compound 5, can be carried out in several different ways. Using a protected derivative of compound 5 for example the trimethylsilylethoxymethylester 5a or the benzhydryl ester 5b the reaction can be carried out using 1-ethoxycarbonyl-2-ethoxy-1,3-dihydroquinoline to afford the protected derivatives 6a and 6b. Removal of the protecting gropus by trifluoroacetic acid affords the desired intermediate 7. Alternatively, compound 4 can be converted to the acid chloride 4b and condensed with the trimethylsilylester 5c, which is procuced in siti. The usual work-up and purification of this reaction gives compound 7.

Scheme III

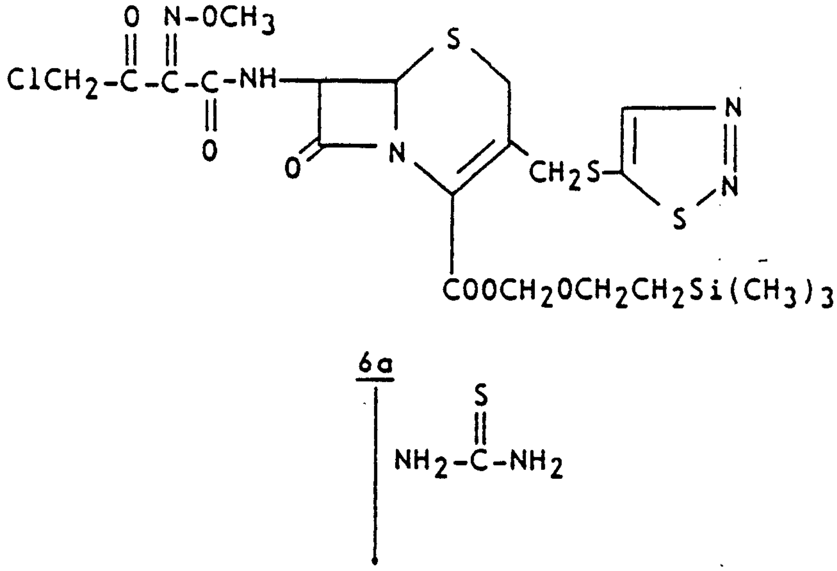

6a

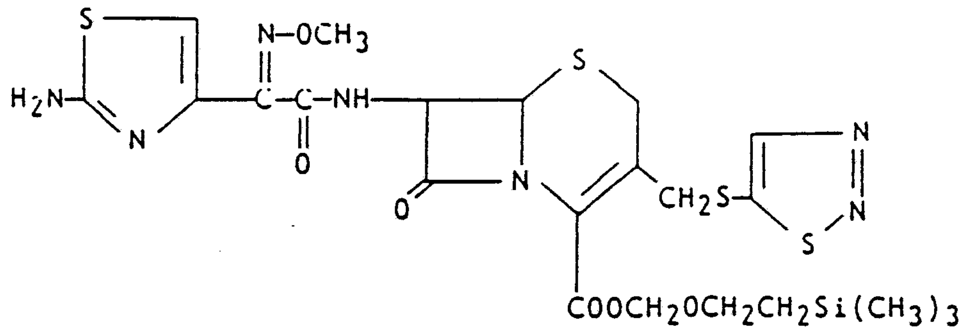

8

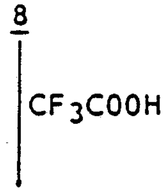


9

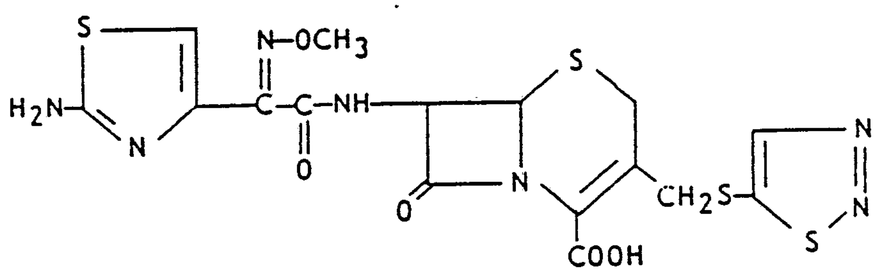

9

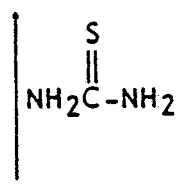

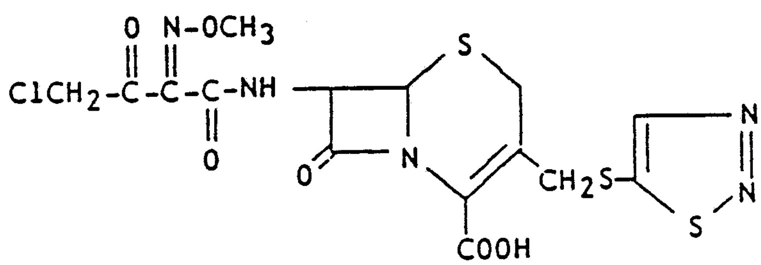

7

The reaction of the protected compound 6a with thiourea gives the trimethylsilylethoxymethyl ester of the cephalosporin 8 which can readily be converted to the desired final product, compound 9 by treatment with trifluoroacetic acid. Similarly compound 9 can be prepared directly by the condensation of compound 7 with thiourea.

Alternatively these reactions can be carried out using radioactive thiourea resulting in a radio-labelled cephalosporin antibiotic.

Preparation of the intermediate compound 7 was first attempted using the method described by Ochiai, et al. J. Antibiotics, 34, 186 (1981) which is illustrated in Scheme IV. Compound 10 (prepared in situ by reaction of diketene with chlorine) was condensed with the 7-aminocephalosporanic acid 5 to afford compound 10 which convertedto the oxime 11 with nitrous acid. Attempted methylation of compound 11 by the usual procedures gave a mixutre of products from which little or none of the desired compound 7 could be isolated. In all probability the complicating factor in this last reaction is the presence of the 1,2,3-thiadiazole moiety in the 3' position of the cephalosporin. Under the reaction conditions this heterocyclic group probably also reacts with the alkylating agent dimethyl sulfate thus accounting for the poor yield of the desired product 7.

Scheme IV

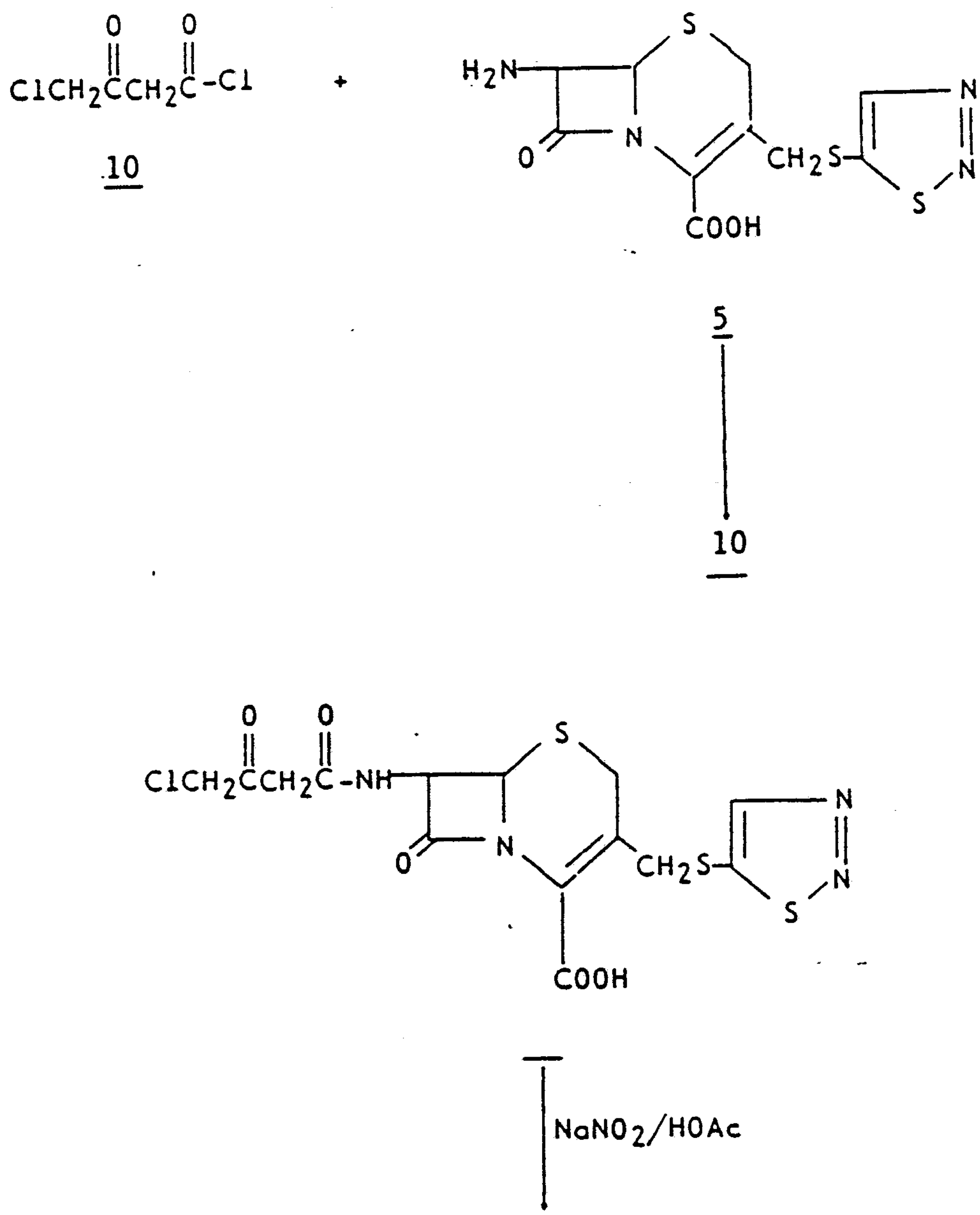


10

5

10

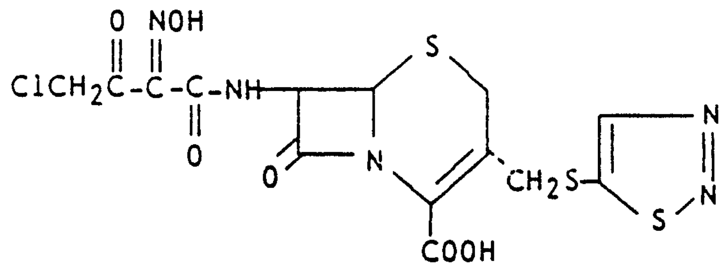

11

$(CH_3)_2SO_4/K_2CO_3$

7

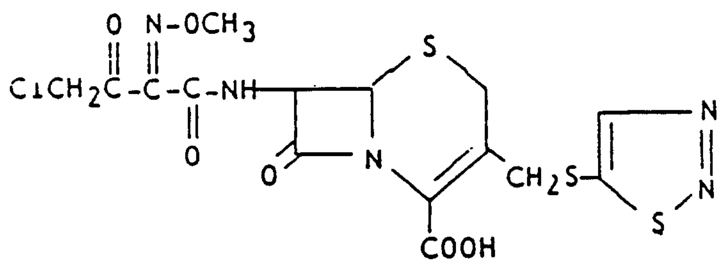

7

An intermediate similar to 4b has been described by Farge, et al. (U. S. Patent 4,307,230) as shown on Scheme V. The disadvantages of this approach are that it is longer and also step 3 is very inefficient, i.e., 52 g of starting oximino ester gave only 8.9 g of the oximino acid.

Scheme V

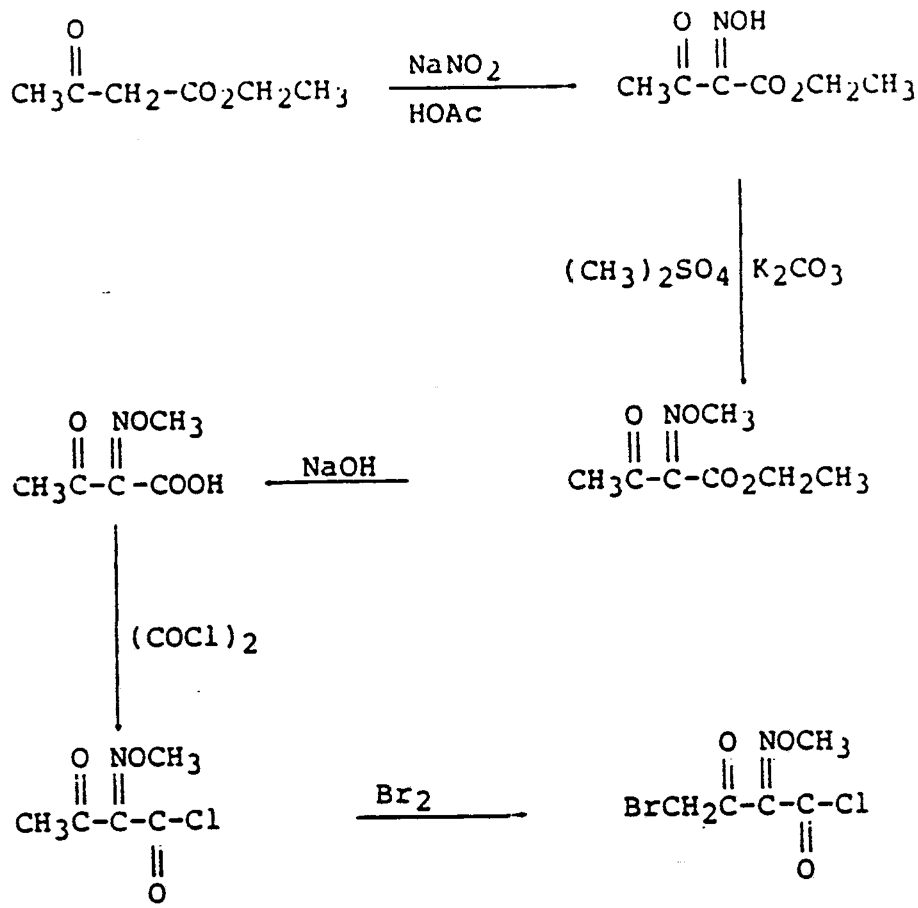


1) A stable crystalline compound 4 was prepared in three steps from a commercially available starting material 1 (Scheme I).

2) This compound can be used to prepare intermediates useful for the preparation of important cephalosporin antibiotics (compound 7 (Scheme II).

The compounds of this invention may be prepared according to the following reaction schemes.

Scheme VI

7-aminocephalosporanic acid, diphenylmethyl ester

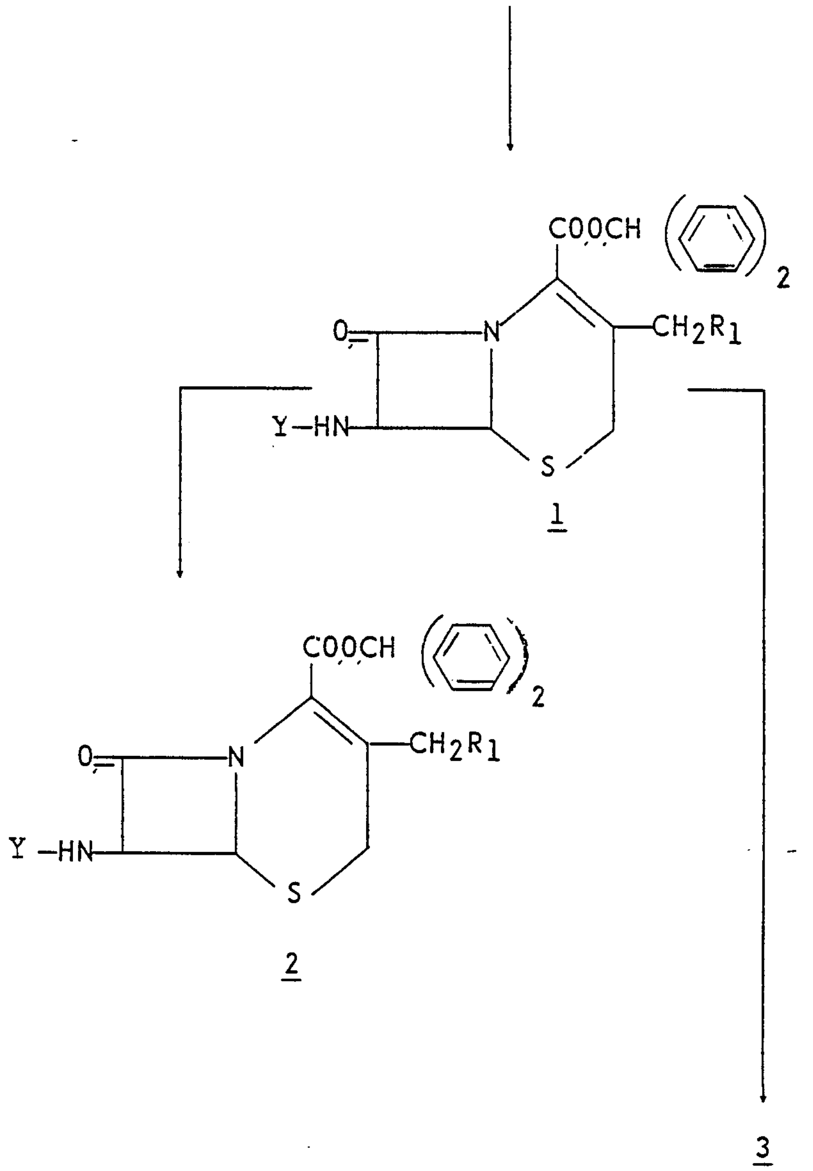

1

2

3

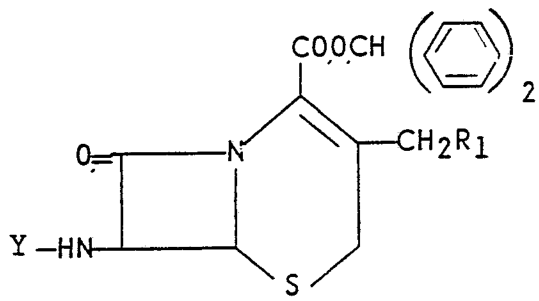

3

According to Scheme VI a 7-aminocephalosporanic acid, diphenylmethyl ester where $R_1$ is as described above is reacted with 2,2,2-trichloroethoxycarbonylisothiocyanate in a solvent such as dichloromethane giving a 3-substituted-8-oxo-7-[[thioxo[[(2,2,2-trichloroethoxy)-carbonyl]amino]methyl]amino]-5-thia-1-azabicyclo[4.2.0]oct-2-ene-2-carboxylic acid, diphenylmethyl ester, 1, where $R_1$ is as described above and Y is $-\overset{S}{\overset{\|}{C}}-NH-\overset{O}{\overset{\|}{C}}-OCH_2CCl_3$. Compound 1 is then reacted with zinc dust, glacial acetic acid and 1 M potassium dihydrogen phosphate in tetrahydrofuran, giving compound 2, a 3-substituted-7-[[[[(2,2-dichloroethoxy)carbonyl]amino]thioxomethyl]amino]-8-oxo-5-thia-1-azabicyclo[4.2.0]oct-2-ene-2-carboxylic acid, diphenylmethyl ester and compound 3, a 3-substituted-7-[(aminothioxomethyl)amino]-8-oxo-5-thia-1-azabicyclo[4.2.0]-oct-2-ene-2-carboxylic acid, diphenylmethyl ester where $R_1$ is as described above and Y is $-\overset{S}{\overset{\|}{C}}-NH_2$ or $-\overset{S}{\overset{\|}{C}}-NH-\overset{O}{\overset{\|}{C}}-OCH_2CHCl_2$, which compounds are separated and purified by chromatography.

Scheme VII

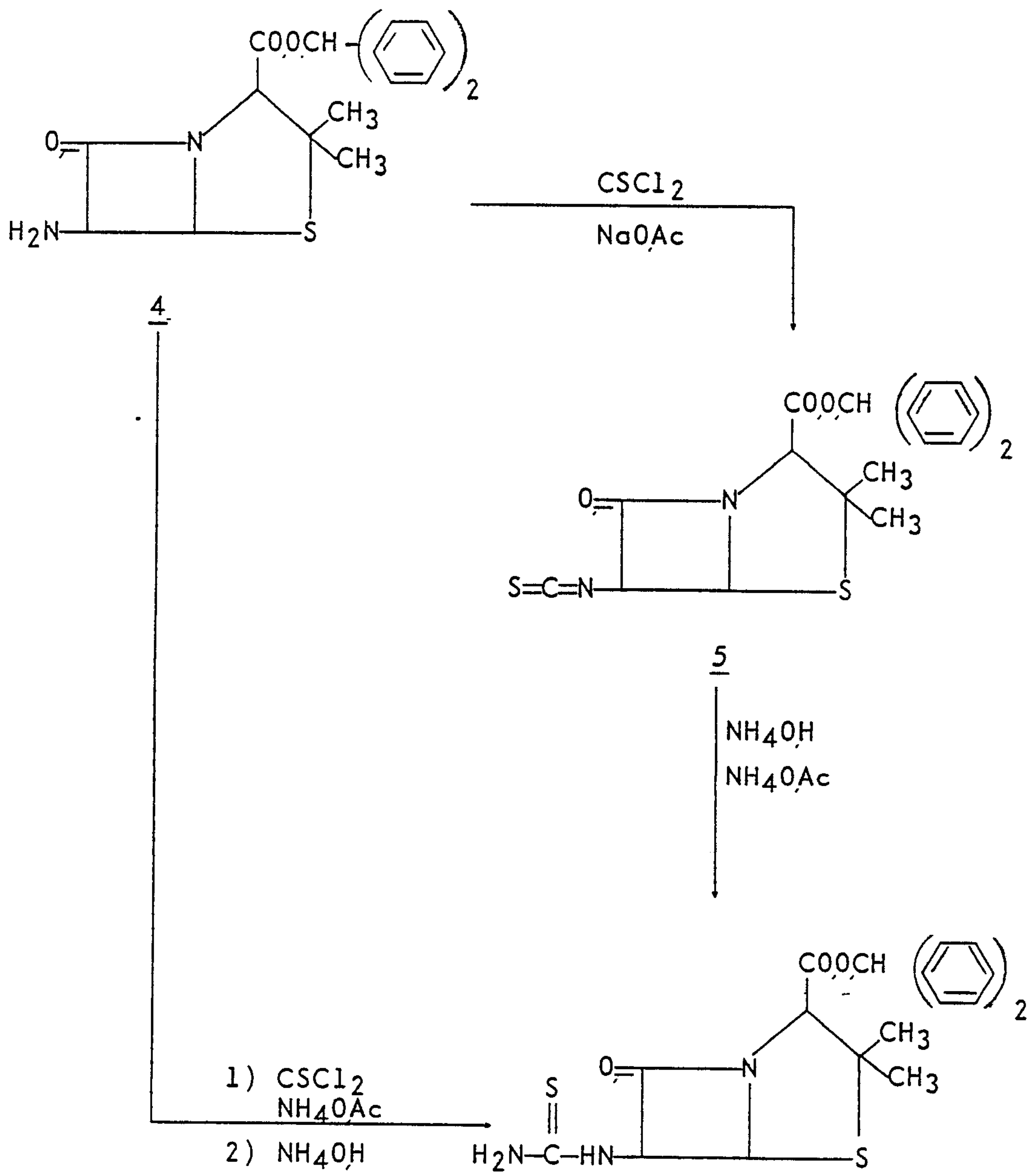


4

5

6

According to Scheme VII, 6-aminopenicillanic acid, diphenylmethyl ester 4 is reacted with thiophosgene and sodium acetate in ethyl acetate giving the isothiocyanate derivative 5, which is then reacted with ammonium acetate in ethyl acetate and water, giving the thiourea derivative 6.

Alternatively 4 may be converted directly to 6 without isolation of 5 by reacting 4 as described above and then adding ammonium hydroxide incrementally.

The reaction described in Scheme V may also be used to convert 7-aminocephalosporanic acid, diphenylmethyl esters 1 (Scheme IV) to the corresponding thiourea derivatives 3 (Scheme VI).

The compounds of this invention are then converted to compounds of the formula:

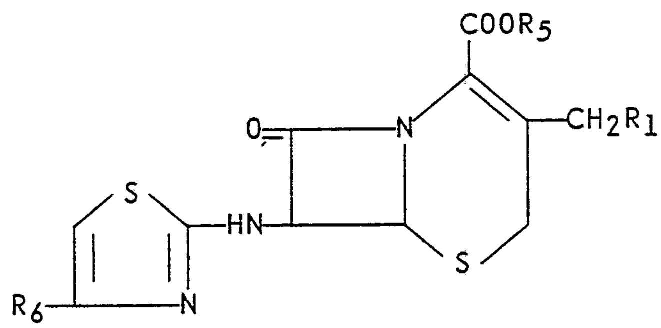

where $R_1$ is as described above, $R_5$ is hydrogen, sodium or potassium and $R_6$ is phenyl, COOH, COOalkyl($C_1$-$C_3$), alkyl-($C_1$-$C_3$) or $COOCH_2CCl_3$, which are active antibacterial agents.

The compounds of this invention may be prepared according to the following reaction scheme:

Scheme VIII

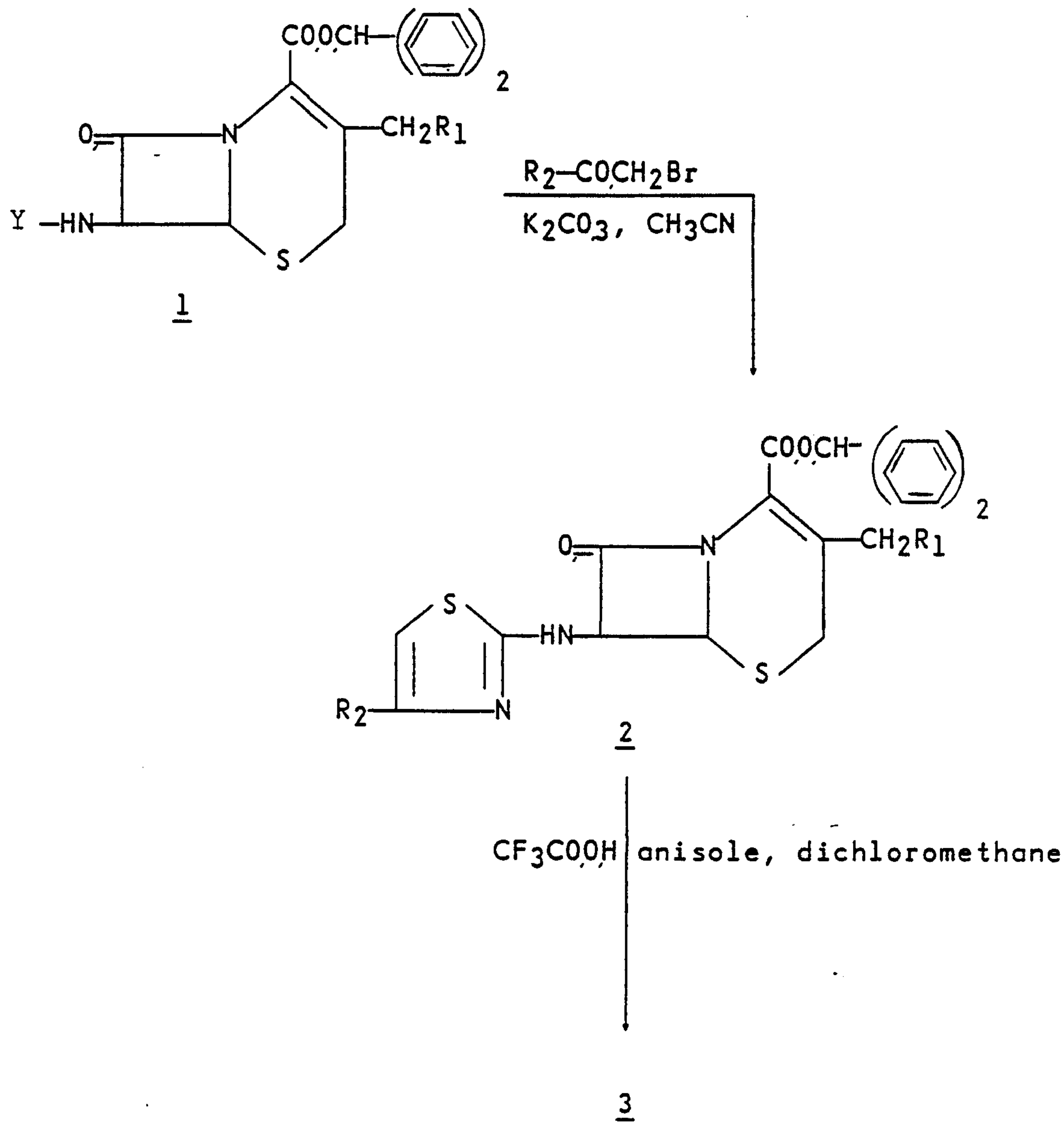


1

2

3

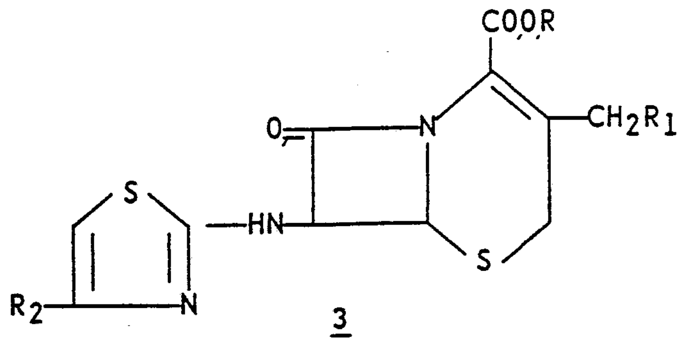

3

According to the above reaction scheme a 3-substituted-8-oxo-7-[(aminothioxomethyl)amino]-5-thia-1-azabicyclo[4.2.0]oct-2-ene-2-carboxylic acid, diphenylmethyl ester 1, where $R_1$ is as described above and

is $-\overset{S}{\overset{\|}{C}}-NH_2$, $-\overset{S}{\overset{\|}{C}}-NH-\overset{O}{\overset{\|}{C}}-OCH_2CCl_3$ or $-\overset{S}{\overset{\|}{C}}-NH-\overset{O}{\overset{\|}{C}}-OCH_2CHCl_2$

is reacted with an α-halogenocarbonyl derivative, where $R_2$ is as described above, and potassium carbonate in acetonitrile, giving diphenylmethyl ester 2 which is then reacted with trifluoroacetic acid and anisole in dichloromethane at 0°C giving the products 3, where $R_1$ and $R_2$ are as described above and R is hydrogen. If R is alkali metal or alkaline earth metal, the products 3 are dissolved in water, to which solution an alkali metal bicarbonate or an alkaline earth metal bicarbonate is added, the resulting mixture then being stirred. The water is evaporated, and the desired antibacterial agents where R is alkali metal or alkaline earth metal are produced.

The compounds of this invention may be prepared according to the following reacting scheme:

Scheme IX

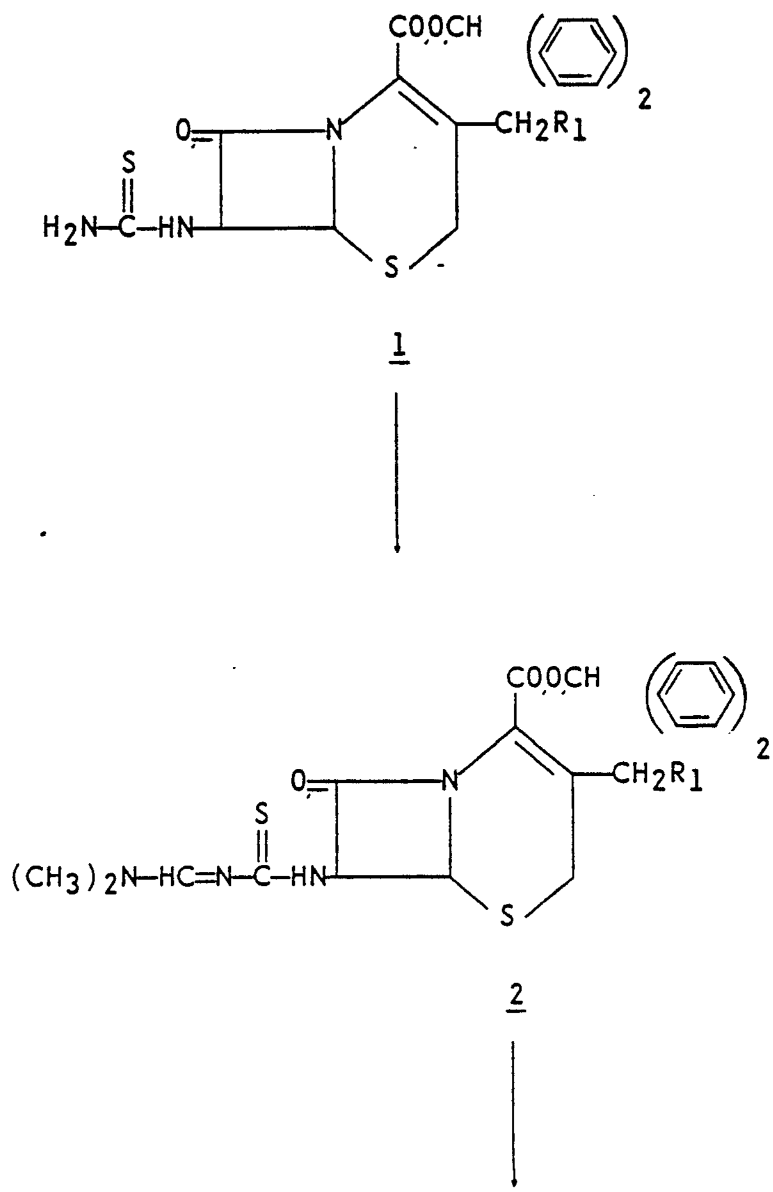

1

2

3

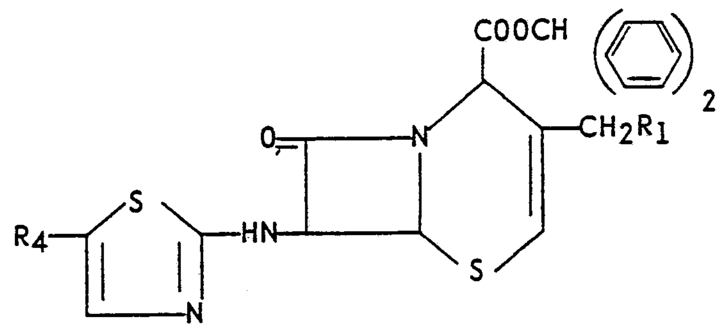

3

According to the above reaction scheme a 3-substituted-7-[(aminothioxomethyl)amino]-8-oxo-5-thia-1-azabicyclo[4.2.0]oct-2-ene-2-carboxylic acid, diphenylmethyl ester 1, where $R_1$ is as described above, is reacted with N,N-dimethylformamide diethylacetal in dichloromethane, giving the 3-substituted-7-[[[[(dimethylamino)-methylene]amino]thioxomethyl]amino]-8-oxo-5-thia-1-aza-bicyclo[4.2.0]oct-2-ene-2-carboxylic acid, diphenylmethyl ester 2, which is reacted with a 2-bromo-substituted ethanone of the formula $R_4COCH_2Br$, where $R_4$ is as described above, and potassium carbonate in acetonitrile, giving the product 3.

The diphenylmethyl ester 3 may be converted to the free carboxylic acid where $R_2$ is hydrogen, by treatment with anisole and trifluoroacetic acid in dichloromethane.

The compounds of this invention where $R_2$ is hydrogen are biologically active and possess antibacterial activity when tested by the Mueller-Hinton agar dilution method against a variety of organisms. The results of this test with representative compounds of this invention appear in Table I.

Compounds of the following formula:

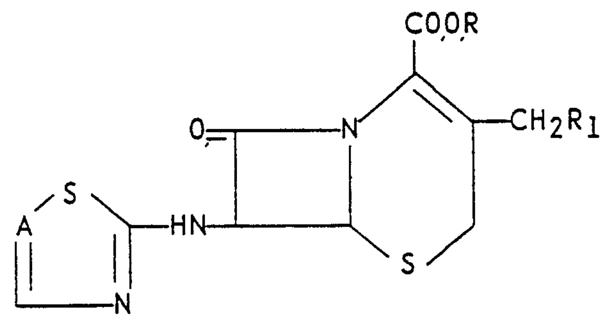

wherein R is hydrogen and $R_1$ and A are as defined above are biologically active and possess antibacterial activity when tested by the Mueller-Hinton agar dilution method against a variety of organisms. The results of this test with representative compounds of this invention appear in Table I.

TABLE I

In vitro Antibacterial Activity

| Organism | | Minimal Inhibitory Concentration (mcg/ml) | |
|---|---|---|---|
| | | Compound of Example No. | |
| | | 34 | 35 |
| Escherichia coli | CMC 84-11 | >128 | >128 |
| Escherichia coli | 311 | >128 | >128 |
| Escherichia coli | ATCC 25922 | >128 | >128 |
| Klebsiella pneumoniae | CMC 84-5 | 128 | >128 |
| Klebsiella pneumoniae | AD | 32 | 128 |
| Klebsiella oxytoca | IO 83-1 | >128 | >128 |
| Enterobacter cloacae | CMC 84-4 | >128 | >128 |
| Enterobacter aeruginosa | IO 83-44 | >128 | >128 |
| Serratia marcescens | CMC 83-27 | >128 | >128 |
| Serratia marcescens | F-35 | >128 | >128 |

TABLE I (continued)

| Organism | | Minimal Inhibitory Concentration (mcg/ml) | |
|---|---|---|---|
| | | Compound of Example No. | |
| | | 34 | 35 |
| Proteus rettgherii | IO 83-21 | >128 | >128 |
| Morganella morganii | IO 83-18 | >128 | >128 |
| Providencia stuartii | CMC 83-82 | >128 | >128 |
| Citrobacter diversis | K 82-24 | 128 | >128 |
| Citrobacter freundii | IO 83-13 | >128 | >128 |
| Acinetobacter | CMC 83-89 | >128 | >128 |
| Acinetobacter | IO 83-49 | >128 | >128 |
| Pseudomonas aeruginsa | CMC 83-19 | >128 | >128 |
| Pseudomonas aeruginsa | 12-4-4 | >128 | >128 |
| Pseudomonas aeruginosa | ATCC 27853 | >128 | >128 |
| Staphylococcus aureus | SSC 82-31 | 1 | 0.12 |
| Staphylococcus aureus | ATCC 25923 | 1 | 0.12 |

TABLE I (continued)

| Organism | | Minimal Inhibitory Concentration (mcg/ml) | |
|---|---|---|---|
| | | Compound of Example No. | |
| | | 34 | 35 |
| *Staphylococcus aureus* | SSC 82-20 | 4 | 1 |
| *Staphylococcus aureus* | SSC 82-26 | 2 | 1 |
| *Staphylococcus aureus* | SSC 82-24 | 128 | 64 |
| *Staphylococcus aureus* | SSC 82-57 | 128 | 128 |
| *Staphylococcus epidermidis* | CMC 83-133 | 1 | 0.12 |
| *Staphylococcus epidermidis* | ATCC 12228 | 2 | 1 |
| *Enterococcus* | CMC 83-53 | 128 | 32 |
| *Streptococcus faecalis* | ATCC 29212 | 64 | 16 |
| *Micrococcus lutea* | PCI 1001 | 2 | 1 |
| *Bacillus subtilis* | ATCC 6633 | 0.25 | 0.5 |

The compounds of the following formula:

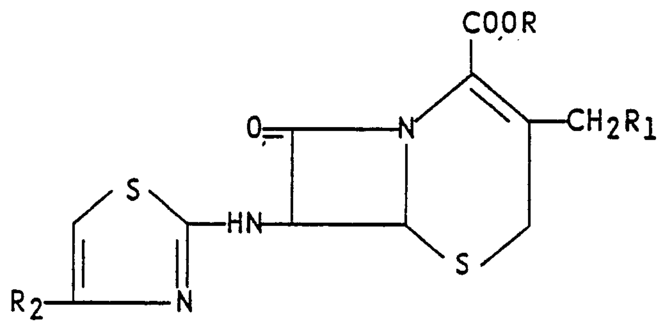

wherein R is hydrogen and $R_1$ and $R_2$ are as defined above are also biologically active and possess potent antibacterial activity when tested by the Mueller-Hinton agar dilution method against a variety of organisms. The results of this test on representative compounds of this invention appear in Table II.

TABLE II

In vitro Antibacterial Activity

| Organism | | Minimal Inhibitory Concentration (mcg/ml) | | | | | |
|---|---|---|---|---|---|---|---|
| | | Compound of Example No. | | | | | |
| | | No. 19 | No. 20 | No. 24 | No. 26 | No. 28 | No. 29 |
| *Candida albicans* | CA 300 | >512 | >512 | - | - | - | - |
| *Saccharomyces* | Y 15 | 128 | 256 | - | - | - | - |
| *Mycobacterium smegmatis* | ATCC 607 | 128 | 256 | - | - | - | - |
| *Bacillus subtilis* | ATCC 6633 | 4 | 8 | 8 | 0.5 | 8 | 1 |
| *Bacillus cereus* | LL No. 4 | 64 | 128 | - | - | - | - |
| *Enterococcus* | OSU 75-1 | 128 | 256 | - | - | - | - |
| *Enterococcus* | CMC 83-53 | - | - | >128 | 64 | >128 | 128 |
| *Enterococcus* | SM 77-15 | 128 | 256 | - | - | - | - |
| *Streptococcus faecalis* | ATCC 29212 | 128 | 256 | >128 | 16 | >128 | 128 |
| *Streptococcus mutans* | ATCC 27352-1 | $\leq 0.25$ | 8 | - | - | - | - |

TABLE II (continued)

| Organism | | Minimal Inhibitory Concentration (mcg/ml) | | | | | |
|---|---|---|---|---|---|---|---|
| | | Compound of Example No. | | | | | |
| | | No. 19 | No. 20 | No. 24 | No. 26 | No. 28 | No. 29 |
| *Streptococcus mutans* | BHI (b) | ≤0.25 | 0.5 | - | - | - | - |
| *Streptococcus sanguis* | G-9B (a) | 4 | 1 | - | - | - | - |
| *Staphylococcus epidermidis* | CMC 83-56 | 0.5 | 8 | - | - | - | - |
| *Staphylococcus epidermidis* | CMC 83-133 | - | - | 2 | ≤0.06 | 2 | 2 |
| *Staphylococcus epidermidis* | ATCC 12228 | 0.5 | 16 | 4 | ≤0.06 | 2 | 2 |
| *Staphylococcus aureus* | Smith | 1 | 8 | - | - | - | - |
| *Staphylococcus aureus* | LL No. 14 | 1 | 4 | - | - | - | - |
| *Staphylococcus aureus* | LL No. 27 | 4 | 16 | - | - | - | - |
| *Staphylococcus aureus* | LL No. 45 | 1 | 4 | - | - | - | - |
| *Staphylococcus aureus* | ATCC 25923 | 1 | 8 | 8 | 0.12 | 4 | 4 |
| *Staphylococcus aureus* | SSC 82-31 | - | - | 8 | ≤0.06 | 4 | 4 |
| *Staphylococcus aureus* | SSC 82-20 | - | - | 16 | 0.25 | 8 | 4 |

TABLE II (continued)

| Organism | | Minimal Inhibitory Concentration (mcg/ml) | | | | | |
|---|---|---|---|---|---|---|---|
| | | Compound of Example No. | | | | | |
| | | No. 19 | No. 20 | No. 24 | No. 26 | No. 28 | No. 29 |
| *Staphylococcus aureus* | SSC 82-26 | - | - | 8 | 0.12 | 4 | 4 |
| *Micrococcus lutea* | PC 1001 | 4 | 8 | 16 | 1 | 8 | 4 |
| *Staphylococcus aureus* | SSC 82-24 | - | - | > 128 | > 128 | > 128 | 64 |
| *Staphylococcus aureus* | SSC 82-57 | - | - | 128 | 128 | 128 | 128 |
| *Escherichia coli* | 311 | 512 | > 512 | > 128 | > 128 | > 128 | 64 |
| *Escherichia coli* | ATCC 25922 | > 512 | > 512 | > 128 | > 128 | > 128 | > 128 |
| *Escherichia coli* | CMC 84-11 | - | - | > 128 | > 128 | > 128 | > 128 |
| *Klebsiella pneumoniae* | AD | 512 | > 512 | > 128 | > 128 | > 128 | 64 |
| *Klebsiella pneumoniae* | CMC 84-5 | - | - | > 128 | > 128 | > 128 | 128 |
| *Klebsiella oxytoca* | IO 83-1 | - | - | > 128 | > 128 | > 128 | > 128 |
| *Enterobacter cloacae* | K79-16 | > 512 | > 512 | - | - | - | - |
| *Enterobacter cloacae* | CMC 84-4 | - | - | > 128 | > 128 | > 128 | > 128 |

TABLE II(continued)

| Organism | | Minimal Inhibitory Concentration (mcg/ml) | | | | | |
|---|---|---|---|---|---|---|---|
| | | Compound of Example No. | | | | | |
| | | No. 19 | No. 20 | No. 24 | No. 26 | No. 28 | No. 29 |
| *Enterobacter aerogenes* | IO 83-44 | - | - | >128 | >128 | >128 | >128 |
| *Serratia* | Tul 78-15 | >512 | >512 | - | - | - | - |
| *Serratia marcescens* | CMC 83-27 | - | - | >128 | >128 | >128 | >128 |
| *Serratia marcescens* | F-35 | - | - | >128 | >128 | >128 | >128 |
| *Salmonella* | QHC 77-3 | >512 | >512 | - | - | - | - |
| *Proteus morganii* | K79-25 | >512 | >512 | - | - | - | - |
| *Citrobacter freundii* | K81-28 | 512 | >512 | - | - | - | - |
| *Citrobacter freundii* | IO 83-13 | - | - | >128 | >128 | >128 | >128 |
| *Acinetobacter* | STFD 79-17 | >512 | >512 | - | - | - | - |
| *Acinetobacter* | CMC 83-89 | - | - | >128 | >128 | >128 | >128 |
| *Acinetobacter* | IO 83-49 | - | - | >128 | >128 | >128 | >128 |
| *Pseudomonas aeruginosa* | 12-4-4 | >512 | >512 | >128 | >128 | >128 | >128 |

TABLE II (continued)

| Organism | | Minimal Inhibitory Concentration (mcg/ml) | | | | | |
|---|---|---|---|---|---|---|---|
| | | Compound of Example No. | | | | | |
| | | No. 19 | No. 20 | No. 24 | No. 26 | No. 28 | No. 29 |
| *Pseudomonas aeruginosa* | ATCC 27853 | >512 | >512 | >128 | >128 | >128 | >128 |
| *Pseudonmonas aeruginosa* | CMC 83-19 | - | - | >128 | >128 | >128 | >128 |
| *Proteus rettgheri* | IO 83-21 | - | - | 128 | 64 | >128 | >128 |
| *Morganella morganii* | IO 83-18 | - | - | >128 | >128 | >128 | >128 |
| *Providencia stuartii* | CMC 83-82 | - | - | >128 | >128 | >128 | >128 |
| *Citrobacter diversis* | K83-24 | - | - | >128 | >128 | >128 | >128 |

The following examples illustrate the preparation of the novel intermediate compounds.

Example 1

t-Butyl 4-chloro-2-(Z)-hydroxyimino-3-oxobutanoate

A solution of 35.6 g of sodium nitrite in 100 ml of water was added to a cold solution of t-butyl 4- chloro-3-oxobutanoate (100.9 g, 0.439 moles of 84% material*) inin 400 ml of acetic acid containing 50 ml of water over a 0.5 hour period with stirring. The mixture was stirred in the cold for an additional hour, then stored in the cold overnight. The mixture was diluted with 500 ml portions of water, 500 ml of saturated sodium bicarbonate, 500 ml of water and 500 ml of brine, then dried over magnesium sulfate and evaporated to give an amber oil; yield 88.6 g (91% yield). This material was used in the next step without further purification.

Example 2

t-Butyl 4-chloro-2-(Z)-methoximino-3-oxobutanoate

Dimethyl sulfate (35.0 ml, 0.370 mol.) was added dropwise over a one hour period to a cold, stirred mixture of t-butyl 4-chloro-2-(Z)-hydroxyimino-3-oxobutanoate (85.7 g, 0.386 mol.) and potassium carbonate (71.5 g, 0.518 mol.) in 500 ml of dry acetone. The mixture was stirred for an additional 3.0 hours in the cold, poured into 500 ml of water and extracted with two 500 ml aliquots of ethyl acetate. The combined ethyl acetate extracts were washed with water and brine then dried over magnesium sulfate. Evaporation of the ethyl acetate at reduced pressure afforded an oil which was purified by flash chromatography on silica gel 60 using ethyl acetate:hexane (1:4) as the eluent to give 44.4 g (51%) of an oil which crystallized after standing at room temperature for several days: IR (neat, $cm^{-1}$) 1720 and 1740 (C=0): NMR ($CDCL_3$) 1.54 [S, 9H, $-C(CH_3)_3$], 4.11 (S, 3H, $-OCH_3$), 4.58 (S, 2H, $-CH_2-$). Anal: Calcd. for $C_9H_{14}No_4Cl$: C, 45.87; H, 5.99; N, 5.94; Cl, 15.04. Found: C. 45.25; H, 5.95; N, 5.98; Cl, 15.26.

*This compound was obtained from Lonza Chemical Co., Fair Lawn, New Jersey.

## Example 3

### 4-Chloro-2-(Z)-methoxyimino-3-oxobutanoic acid

A solution of t-butyl 4-chloro-2-(Z)-methoxyimino-3-oxobutanoate (8.65 g, 0.0367 mol.) in 50 ml of methylene chloride and 50 ml of trifluoroacetic acid was stirred at room temperature for 4 hours. Added 15 ml more of trifluoroacetic acid and stirred for an additional 1.25 hours, then evaporated to dryness at reduced pressure (30°C). The residue was dissolved in 50 ml of methylene chloride, evaporated (30°C) and the residue was crystallized using methylene chloride:hexane to give 4.40 g (67%) of a crystalline solid, mp 91-93°C: IR (KBr, $cm^{-1}$) 1705 and 1725 (C=O): NMR ($CDCl_3$) δ 4.20 (S, 3H, $-OCH_3$), 4.63 (S, 2H, $-CH_2-$), 11.11 (S, 1H, -OH). Anal: Calcd. for $C_5H_6NO_4Cl$: C, 33.51; H, 3.38; N, 7.81; Cl, 19.81. Found: C, 33.21; H, 3.16; N, 7.90; Cl, 19.63.

## Example 4

### 7β-[4-Chloro-2-(Z)-methoxyimino-3-oxobutyramido]-3-[(1,2,3-thiadiazol-5-yl)thiomethyl]ceph-3-em-4-carboxylic acid

Method A

Oxalyl chloride (0.87 ml, 10 mmol.) was added dropwise to an ice cold solution of 4-chloro-2-(Z)-methoxyimino-3-oxobutanoic acid (1.79 g, 10 mmol.) and pyridine (0.81 ml, 10 mmol.) in 50 ml of methylene chloride. The mixture was stirred in the cold for 15 minutes, at room temperature for 15 minutes, then evaporated to dryness at reduced pressure (30°C). The residue was dissolved in 50 ml of methylene chloride and added, over 30 minutes, to an ice cold (ice/methanol bath) solution of 7-amino-3-[(1,2,3-thiadiazol-5-yl)thiomethyl]-ceph-3-em-4-carboxylic acid (3.30 g, 10 mmol.) and bis trimethylsilylacetamide (10 ml, 40.5 mmol.) in 100 ml of ethyl acetate (which had previously been stirred at room temperature for 6 hours).

The mixture was stirred in the cold for an additional 15 minutes, then at room temperature for 30 minutes, and poured into a mixture of 100 ml of ethyl acetate and 50 ml of water. The resulting mixture was filtered to remove some insoluble material, the filtrate was separated and the ethyl acetate layer was extracted with four 100 ml aliquots of water then dried over magnesium sulfate. The ethyl acetate was filtered through hydrous magnesium silicate and then stirred with 150 ml of 0.2N sodium bicarbonate solution. The layers were separated and the organic phase was re-extracted with 50 ml of 0.2N sodium bicarbonate solution. The combined aqueous layers were acidified to pH 2.2 with 4N hydrochloric acid and extracted with two 100 ml portions of ethyl acetate. The ethyl acetate solution was washed with water and brine then dried over magnesium sulfate. Evaporation of ethyl acetate afforded the desired product (1.64 g) as a light yellow glass. IR (KBr, $cm^{-1}$) 1780, 1755, and 1675; NMR (DMSO-$d_6$) $\delta$ 3.70 (pair of doublets, 2H, J = 17.9Hz, endocyclic $-CH_2S-$), 4.05 (S, 3H, $-OCH_3$), 4.25 (pair of doublets, 2H, J = 15Hz, exocyclic $-CH_2-S-$), 4.85 (S, 2H, $ClCH_2-$), 5.20 (d, 1H, J = 3.7Hz, 6H of 7ACA), 5.80 (double doublet, 1H, J = 3.7 and 8.4Hz, 7H of 7ACA), 8.89 (S, 1H, thiadiazole H), 9.48 (d, 1H, J = 8.4Hz, -NH).

Method B

2-(Trimethylsilyl)ethoxymethyl 7β-[4-chloro-2-(Z)-methoxyimino-3-oxobutyramido-3-[(1,2,3-thiadiazol-5-yl)thiomethyl]ceph-3-em-carboxylate (1.5 g, 2.41 mmol.) was stirred in 25 ml of trifluoroacetic acid for 1.0 hour at room temperature. The solution was evaporated to dryness at 40°C and the residue disolved in methylene chloride and again evaporated at reduced pressure. The residue was partitoned in 75 ml of ethyl acetate and 150 ml of 0.2N sodium bicarbonate solution. The layers were separated and the ethyl acetate was extracted with 25 ml more of 0.2N sodium bicarbonate solution. The combined aqueous

portions were acidified to pH 2.5 and extracted with 75 ml aliquots of ethyl acetate. The combined ethyl acetate extracts were washed with brine, dried over magnesium sulfate and evaporated to give 0.83 g (75%) of a yellow glass.

Method C

Diphenylmethyl 7 -[4-chloro-2-(Z)-methoxyimino-3-oxobutyramido]-3-[(1,2,3-thiadiazol-5-yl)thiomethyl]ceph-3-em-4-carboxylate (3.25 g, 4.94 mmol.) was treated in the same manner as described in Method B to afford 2.11 g (87%) of the desire product.

Example 5

2-(Trimethylsilyl)ethoxymethyl 7-amino-3-[(1,2,3-thiadiazol-5-yl)thiomethyl]ceph-3-em-4-carboxylate

A mixture of 7-amino-3-[(1,2,3-thiadiazol-5-yl)-thiomethyl]ceph-3-em-4-carboxylic acid (3.30 g, 10 mmol.) and triethylamine (1.40 ml, 10 mmol.) in 50 ml of dimethylformamide was stirred at room temperature for 1.0 hour. Trimethylsilylethoxymethyl chloride (1.80 ml, 10 mmol.) was added dropwise, the reaction mixture was stirred for 15 minutes at room temperature then poured into 100 ml of water and extracted with 80 ml of ethyl acetate. The ethyl acetate extract was washed with 75 ml aliquots of saturated sodium bicarbonate, water and brine then dried over magnesium sulfate. Evaporation of the solvent afforded 3.05 g of product as an amber oil: NMR ($CDCl_3$) $\delta$ 0.03 [S, 9H, $Si(CH_3)_3$], 0.95 (t, 2H, J = 7.5Hz, $-CH_2Si$), 2.00 (S, 2H, $NH_2$), 3.50 (m, 4H, $-OCH_2Si$ and indocyclic $-CH_2-S-$), 4.00 (S, 2H, exocyclic $-CH_2S-$), 4.60 (d, 1H, J = 5.0Hz, 6H of 7ACA), 4.80 (d, 1H, J = 5.0Hz, 7H of 7ACA), 5.30 (S, 2H, $-OCH_2O-$), 8.40 (S, 1H, thiadiazole H).

## Example 6

**Diphenylmethyl 7β-[4-chloro-2-(Z)-methoxyimino-3-oxobutyramido]-3-[(1,2,3-thiadiazol-5-yl)thiomethyl]-ceph-3-em-4-carboxylate**

A solution of 4-chloro-2-(Z)-methoxyimino-3-oxo-butanoic acid (4.05 g, 22.6 mmol.), diphenylmethyl 7-amino-3-[(1,2,3-thiadiazol-5-yl)thiomethyl]ceph-3-em-4-carboxylate (11.2 g, 22.6 mmol.) and 1-ethoxycarbonyl-2-ethoxy-1,2-dihydroquinoline (5.58 g, 22.6 mmol.) in 200 ml of methylene chloride was stirred at room temperature for 3 hours. The solution was evaporated to dryness at reduced pressure and the residue was taken up in 150 ml of ethyl acetate which was extracted with 50 ml aliquots of 1N hydrochloric acid, water, saturated sodium bicarbonate, water and brine. The ethyl acetate was dried over magnesium sulfate, evaporated to dryness and the resulting product was purified by flash chromatography on silica gel 60 using ethyl acetate: hexane (1:2) as the eluent to afford 3.34 g of the desired compound:NMR ($CDCl_3$) δ 3.52 (pair of d, 2H, J = 18Hz, endocyclic $-CH_2S-$), 4.05 (pair of d, 2H, J = 13Hz, exocyclic $-CH_2S-$), 4.15 (S, 3H, $-OCH_3$)j, 4.59 (S, 2H, $ClCH_2-$), 5.04 (d, 1H, J = 4.7Hz, 6H of 7ACA), 5.88 (dd, 1H, J = 4.7 and 8.6Hz, 7H of 7ACA), 6.89 (S, 1H, -CH-), 7.33 (m, 10H, aromatic H), 8.37 (S, 1H, thiadiazole H).

## Example 7

**2-(Trimethylsilyl)ethoxymethyl 7β-[4-chloro-2-(Z)-methoxyimino-3-oxobutyramido]-3-[(1,2,3-thiadiazol-5-yl)thiomethyl]ceph-3-em-4-carboxylate**

A solution of 4-chloro-2-(Z)-methoxyimino-3-oxo-butanoic acid (2.17 g, 4.77 mmol.), 2-(trimethylsilyl)-ethoxymethyl 7-amino-3-[(1,2,3-thiadiazol-5-yl)thiomethyl]-ceph-3-em-4-carboxylate (0.85 g, 4.77 mmol.), and 1-ethoxy-carbonyl-2-ethoxy-1,2-dihydroquinoline (1.18 g, 4.77 mmol.) in 40 ml of methylene chloride was stirred at room temperature for 3 hours then worked up as described in Example 6 to afford 0.615 g of the ester. NMR ($CDCl_3$) δ 0.03 [S,

9H, $-Si(CH_3)_3]$, 0.97 (t, 2H, J = 7.5Hz, $-CH_2Si-$), 3.62 (pair of d, 2H, J = 18Hz, endocyclic $-CH_2S-$), 3.75 (m, 2H, $-OCH_2-$), 4.17 (pair of d, 2H, J = 13.3Hz, exocyclic $-CH_2S$), 4.18 (S, 3H, $-OCH_3$), 4.63 (S, 2H, $-ClCH_2-$), 5.07 (d, 1H, J = 4.9, 6H of 7ACA), 5.38 (pair of d, 2H, J = 6.0Hz, $-OCH_2O$), 5.87 (dd, 1H, J = 4.9 and 8.5Hz, 7H of 7ACA), 7.22 (d, 1H, J = 8.5Hz, -NH-), 8.52 (S, 1H, thiadiazole H).

## Example 8

## 2-(Trimethylsilyl)ethoxymethyl 7β-[2-(2-aminothiazol-4-yl)-(Z)-2-methoxyiminoacetamido]-3-[(1,2,3-thiadiazol-5-yl)thiomethyl]ceph-3-em-4-carboxylate

Method A

A solution of 2-(trimethylsilyl)ethoxymethyl 7β-[4-chloro- 2-(Z)-methoxyimino-3-oxobutyramido]-3-[(1,2,3-thiadiazol-5-yl)thiomethyl]ceph-3-em-4-carboxylate (622 mg, 1 mmol.) and thiourea (76 mg, 1 mmol.) in 8 ml of dimethylacetamide was stirred at room temperature overnight. The reaction mixture was diluted with 50 ml of ethyl acetate and extracted with dilute sodium bicarbonate, water and brine. The ethyl acetate solution was dried over magnesium sulfate, evaporated at reduced pressure, and the resulting product was purified by preparative thick layer chromatography on silica gel using ethyl acetate:hexane (2:1) as the eluent to afford 124 mg of the desired product: NMR ($DMSO-d_6$ + $CDCl_3$) δ 0.03 [S, 9H, $-Si(CH_3)_3]$, 0.96 (t, 2H, J = 7.5Hz, $-CH_2Si-$), 3.62 (S, 2H, endocyclic $-CH_2S-$), 3.74 (t, 2H, J = 7.5Hz, $-OCH_2Si-$), 4.02 (S, 3H, $-OCH_3$), 4.15 (pair of doublets, 2H, J = 13.4Hz, exocyclic $-CH_2-S$), 5.12 (d, 1H, J = 4.7Hz, 6H of 7ACA), 5.37 (m, 2H, $-OCH_2O$), 5.90 (double doublet, 1H, J = 8.4 and 4.7Hz, 7H of 7ACA), 6.15 (S, 2H, $-NH_2$), 6.80 (S, 1H, thiadiazole H), 8.55 (S, 1H, thiadiazole H), 9.15 (d, 1H, J = 8.4Hz, -NH-).

Method B

A solution of sodium 7β-[2-(2-aminothiazol-4-yl)-(Z)- 2-methoxyiminoacetamido]-3-[(1,2,3-thiadiazol-5-yl)-thiomethyl]ceph-3-em-4-carboxylate (535 mg, 1 mmol.) and 2-(trimethylsilyl)ethoxymethyl chloride (0.18 ml, 1 mmol.) in 5 ml of dimethylformamide was stirred at room temperature for 5 minutes. The mixture was diluted with 50 ml of ethyl acetate and extracted with dilute sodium bicarbonate, water and brine, then dried over magnesium sulfate. Evaporation of the solvent afforded 535 mg of product. The infrared and nuclear magnetic resonance spectra were the same as the material described in Method A.

Example 9

7β-[2-(2-Aminothiazol-4-yl)-(Z)-2-methoxyimino-acetamido]-3-[(1,2,3-thiadiazol-5-yl)thiomethyl]-ceph-3-em-4-carboxylic acid

A solution of 7β-[4-chloro-2-(Z)-methoxyimino-3-oxobutyramido]-3-[(1,2,3-thiadiazol-5-yl)thiomethyl]ceph-3-em-4-carboxylic acid (254 mg, 0.52 mmol.) and thiourea (80 mg, 1.05 mmol.) in 8 ml of dimethylacetamide was stirred at room temperature overnight then poured into 20 ml of ice water. The pH was adjusted to 3.5 with dilute sodium bicarbonate and the product was collected by filtration, yield 125 mg.

The following examples illustrate the preparation of the final products.

Example 10

(6-R-trans)-3-[(Acetyloxy)methyl]-8-oxo-7-[[thioxo[[(2,2,2-trichloroethoxy)carbonyl]-amino]methyl]amino]-5-thia-1-azabicyclo-[4.2.0]oct-2-ene-2-carboxylic acid, diphenylmethyl ester

A mixture 4.03 g of carbon(isothiocyanatidic) acid, 2,2,2-trichloroethyl ester, 7.49 g of (6R-trans)-3-[(acetyloxy)methyl-7-amino-8-oxo-5-thia-1-azabicyclo[4.2.0]-oct-2-ene-2-carboxylic acid, diphenylmethyl ester and 125 ml of dichloromethane was stirred for 30 minutes, then filtered through hydrous magnesium silicate and evaporated giving 11.47 g of the desired compound $[\alpha]_D^{26}=+29^o\pm2^o$ (c 0.51% $CHCl_3$).

Example 11

(6R-trans)-3-[(Acetyloxy)methyl]-7-[(amino-thioxomethyl)amino]-8-oxo-5-thia-1-aza-bicyclo[4.2.0]oct-2-ene-2-carboxylic acid, diphenylmethyl ester and (6R-trans)-3-[(acetyloxy)methyl]-7-[[[[(2,2-dichloroethoxy)carbonyl]amino]-thioxomethyl]amino]-8-oxo-5-thia-1-aza-bicyclo[4.2.0]oct-2-ene-2-carboxylic acid, diphenylmethyl ester

A mixture of 1.16 g of (6R-trans)-3-[(acetyloxy)-methyl]-8-oxo-7-[[thioxo[[(2,2,2-trichloroethoxy)carbonyl]-amino]methyl]amino]-5-thia-1-azabicylco[4.2.0]oct-2-ene-2-carboxylic acid, diphenylmethyl ester, 660 mg of zinc dust, 0.25 ml of glacial acetic acid, 2 ml of 1M aqueous potassium dihydrogen phosphate and 10 ml of tetrahydrofuran was stirred for 1 hour and then filtered. The filtrate was subjected to flash chromatography, eluting with ethyl acetate:petroleum ether in a 1:1 to 1:2 gradient, giving 340 mg of (6R-trans)

-3-[(acetyloxy)methyl]-7-[(aminothioxomethyl)amino]-8-oxo-5-thia-1-azabicyclo[4.2.0]oct- 2-ene-2-carboxylic acid, diphenylmethyl ester and 30 mg of (6R-*trans*)-3-[(acetyloxy) methyl]-7-[[[[(2,2-dichloroethoxy)carbonyl]amino]thioxomethyl]amino]-8-oxo-5-thia-1-azabicyclo[4.2.0]oct-2-ene-2-carboxylic acid, diphenylmethyl ester.

## Example 12

## [2S-(2α,5α,6β)]-3,3-Dimethyl-7-oxo-6-[[thioxo[[(2,2,2-trichloroethoxy)carbonyl]amino]methyl]amino]-4-thia-1-azabicyclo[3.2.0]heptane-2-carboxylic acid, diphenylmethyl ester

A 5.0 ml portion of trichloroethoxy chloroformate was added dropwise to a warm (50°C) solution of potassium thiocyanate in 100 ml acetonitrile. This solution was stirred at 50°C for 20 minutes, then allowed to cool to room temperature and chilled in an ice bath. The resulting solution was filtered and concentrated *in vacuo*. Vacuum distillation of the residue at 70°C, 1mm Hg, gave 7.60 g of 2,2,2-trichloroethoxycarbonylthiocyanate.

A 14.04 g portion of 6-aminopencillanic acid benzhydryl ester pTSOH salt was partitioned between 150 ml of dichloromethane and saturated aqueous sodium bicarbonate. The dichloromethane layer was separated and dried over sodium sulfate. To this solution was added 5.63 g of 2,2,2-trichloroethoxycarbonylthiocyanate. This mixture was stirred for 1 hour, filtered through hydrous magnesium silicate and concentrated, giving 13.3 g of the desired product as a white foam.

## Example 13

## 3,3-Dimethyl-6-isothiocyanato-7-oxo-4-thia-1-azabicyclo[3.2.0]heptane-2-carboxylic acid, diphenylmethyl ester

A 555 mg portion of 6-amino-3,3-dimethyl-7-oxo-4-thia-1-azabicyclo[3.2.0]heptane-2-carboxylic acid, diphenylmethyl ester was dissolved in 20 ml of ethyl acetate. A 400 mg portion of sodium acetate was dissolved in 10 ml of water and

added to the above solution. A 0.09 ml portion of thiophosgene was added, the mixture was stirred 15 minutes and the layers separated. The organic layer was washed with brine, dried and evaporated, giving 447 mg of the desired compound.

Example 14

6-[(Aminothioxomethyl)-3,3-dimethyl-7-oxo-4-thia-1-azabicyclo[3.2.0]hetane-2-carboxylic acid, diphenylmethyl ester

A 447 mg portion of 3,3-dimethyl-6-isothiocyanato-7-oxo-4-thia-1-azabicyclo[3.2.0]heptane-2-carboxylic acid, diphenylmethyl ester was dissolved in 15 ml of ethyl acetate. A solution of 1 g of ammonium acetate in 10 ml of water was added. Six drops of concentrated ammonium hydroxide were added with stirring. Stirring was continued for 1 hour, then the layers were separated. The organic layer was washed twice with brine, dried and evaporated to 5 ml. This was diluted to 100 ml with hexane and chilled. The solid was collected, giving 261 mg of the desired compound.

Example 15

6-[(Aminothioxomethyl)amino]-3,3-dimethyl-7-oxo-4-thia-1-azabicyclo[3.2.0]heptane-2-carboxylic acid, diphenylmethyl ester

A 0.9 ml portion of thiophosgene was added to a stirred mixture of 5.55 g of 6-amino-3,3-dimethyl-7-oxo-4-thia-1-azabicyclo[3.2.0]heptane-2-carboxylic acid, diphenylmethyl ester in 100 ml of ethyl acetate and 5.0 g of ammonium acetate in 50 ml of water. After stirring for 10-15 minutes concentrated ammonium hydroxide was added in increments over 5 hours. The layers were separated and the organic layer washed twice with brine, dried, evaporated to about 20 ml and chilled. The solid was collected, giving 2.385 g of the desired compound.

## Example 16

## 3-[(Acetyloxy)methyl]-7-isothiocyanato-8-oxo-5-thia-1-azabicyclo[4.2.0]oct-2-ene-2-carboxylic acid, diphenylmethyl ester

The procedure of Example 13 was followed using 439 mg of 3-[(acetyloxy)methyl]-7-amino-8-oxo-5-thia-1-azabicyclo[4.2.0]oct-2-ene-2-carboxylic acid, diphenylmethyl ester and 240 mg of sodium acetate, giving 535 mg of the desired compound as a yellow oil.

## Example 17

## (6R-cis)-3-[(Acetyloxy)methyl]-7-[(aminothioxomethyl)amino]-8-oxo-5-thia-1-azabicyclo[4.2.0]oct-2-ene-2-carboxylic acid, diphenylmethyl ester

The procedure of Example 15 was followed using 4.39 g of 3-[(acetyloxy)methyl]-7-amino-8-oxo-5-thia-1-azabicyclo[4.2.0]oct-2-ene-2-carboxylic acid, diphenylmethyl ester. The resulting oil was dissolved in 25 ml of ethyl acetate, hexane was added with warming to enhance solution, then the mixture was allowed to stand for several hours and chilled. The resulting solid was removed by filtration and the filtrate evaporated to a glass. This glass was dissolved in a small amount of ethyl acetate, silica gel was added, the mixture evaporated to dryness and added to the top of a 150 g silica gel column. The column was flash chromatographed eluting as follows:

Fractions 1 to 8 - 8x80 ml of ethyl acetate:hexane (30:70)

Fractions 9 to 20 - 12x80 ml of ethylacetate:hexane (40:60)

Fractions 21 to 24 - 4x100 ml of ethylacetate:hexane (50:50)

Fractions 25 to 34 - 10x100 ml of ethyl acetate: hexane (30:20)

Fraction 31 was evaporated and crystallized from a small amount of ethyl acetate:hexane giving 44 mg of the desired compound as light yellow crystals.

Example 18

(6R-trans)-3-[(Acetyloxy)methyl]-8-oxo-7-[(4-phenyl-2-thiazolyl)amino]-5-thia-1-azabicyclo[4.2.0]oct-2-ene-2-carboxylic acid, diphenylmethyl ester

A mixture of 539 mg of (6R-trans)-3-[(acetyloxy)methyl]-7-[(aminothioxomethyl)amino]-8-oxo-5-thia-1-azabicyclo[4.2.0]oct-2-ene-2-carboxylic acid, diphenylmethyl ester, 240 mg of 2-bromo-1-phenylethanone, 85 mg of potassium carbonate and 7 ml of acetonitrile was stirred overnight, then filtered through hydrous magnesium silicate. The filtrate was evaporated, giving 660 mg of the desired compound $[\alpha]_D^{26}=+68^o\pm3^o$ (c, 0.4 chloroform).

Example 19

(6R-trans)-3-[(Acetyloxy)methyl]-7-[[4-ethoxycarbonyl)-2-thiazolyl]amino]-5-thia-1-azabicyclo[4.2.0]oct-2-ene-2-carboxylic acid, diphenylmethyl ester

A mixture of 450 mg of (6R-trans)-3-[(acetyloxy)methyl]-7-[(aminothioxomethyl)amino]-8-oxo-5-thia-1-azabicyclo[4.2.0]oct-2-ene-2-carboxylic acid, diphenylmethyl ester, 195 mg of 3-bromo-2-oxopropanoic acid, ethyl ester, 70 mg of potassium carbonate and 7 ml of acetonitrile was stirred overnight and then filtered through hydrous magnesium silicate. The filtrate was evaporated, giving 510 mg of (6R-trans)-3-[acetyloxy)methyl]-7-[[4-(

(ethoxycarbonyl)-2-thiazolyl]amino]-8-oxo-5-thia-1-aza-bicyclo[4.2.0]oct-2-ene-2-carboxylic acid, diphenylmethyl ester.

A mixture of 105 mg of the above ester, 0.5 ml of trifluoroacetic acid, 2 drops of anisole and 2 ml of dichloromethane was stirred at 0°C for 30 minutes, then at room temperature for 30 minutes. The addition of ether and petroleum ether gave a white precipitate. The precipitate was washed with ether and petroleum ether, giving 45 mg of the desired product.

Example 20

(6R-trans)-3-[(Acetyloxy)methyl]-8-oxo-7-[(4-phenyl-2-thiazolyl)amino]-5-thia-1-azabicyclo[4.2.0]oct-2-ene-2-carboxylic acid

A mixture of 182 mg of (6R-trans)-3-[(acetyloxy)methyl]-8-oxo-7-[(4-phenyl-2-thiazolyl)amino]-5-thia-1-azabicyclo[4.2.0]oct-2-ene-2-carboxylic acid, diphenylmethyl ester, 0.5 ml of trifluoroacetic acid, 3 drops of anisole and 3 ml of dichloromethane was reacted as described in Example19, giving 92 mg of the desired compound.

Example 21

(6R-trans)-3-[(Acetyloxy)methyl]-7-[(4-methyl-2-thiazolyl)amino]-8-oxo-5-thia-1-azabicyclo[4.2.0]oct-2-ene-2-carboxylic acid, diphenylmethyl ester

A mixture of 91 mg of (6R-trans)-3-[(acetyloxy)methyl]-7-[(aminothioxomethyl)amino]-8-oxo-5-thia-1-azabicyclo[4.2.0]oct-2-ene-2-carboxylic acid, diphenylmethyl ester, 33 mg of sodium iodide, 20 mg of potassium carbonate and 2 ml of acetonitrile was reacted as described in Example 19, giving the desired compound.

Example 22

(6R-trans)-3-[(Acetyloxy)methyl]-8-oxo-7-[[4-[[2-trimethylsilyl)ethoxy]carbonyl]-2-thiazolyl]amino]-5-thia-1-azabicyclo-[4.2.0]oct-2-ene-2-carboxylic acid, diphenylmethyl ester

A mixture of 750 mg of (6R-trans)-3-[(acetyloxy)methyl]-7-[(aminothioxomethyl)amino]-8-oxo-5-thia-1-azabicyclo[4.2.0]oct-2-ene-2-carboxylic acid, diphenylmethyl ester, 470 mg of 3-bromo-2-oxopropanoic acid, trimethylsilyl ethyl ester, 110 mg of potassium carbonate and 15 ml of acetonitrile was reacted as described in Example 18, giving 850 mg of the desired product.

Example 23

(6R-trans)-3-[(Acetyloxy)methyl]-7-[(4-carboxy-2-thiazolyl)amino]-8-oxo-5-thia-1-azabicyclo[4.2.0]oct-2-ene-2-carboxylic acid, diphenylmethyl ester

A mixture of 770 mg of (6R-trans)-3-[(acetyloxy)methyl]-7-[(aminothioxomethyl)amino]-8-oxo-5-thia-1-azabicyclo[4.2.0]oct-2-ene-2-carboxylic acid, diphenylmethyl ester, 425 mg of 3-bromo-2-oxopropanoic acid, trimethylsilyl ester, 110 mg of potassium carbonate and 15 ml of acetonitrile was reacted as described in Example 18, giving 810 mg of the desired compound.

Example 24

(6R-trans)-3-[(Acetyloxy)methyl]-7-[(4-carboxy-2-thiazolyl)amino]-8-oxo-5-thia-1-azabicyclo[4.2.0]oct-2-ene-2-carboxylic acid

A mixture of 300 mg of (6R-trans)-3-[(acetyloxy)methyl]-7-[(4-carboxy-2-thiazolyl)amino]-8-oxo-5-thia-1-azabicyclo[4.2.0]oct-2-ene-2-carboxylic acid, diphenylmethyl ester, 1 ml of trifluoroacetic acid, 0.25 ml of anisole and 3 ml of dichloromethane was allowed to stand for 45 minutes, then the solid was collected, washed with

ether and petroleum ether and dried, giving 155 mg of the desired compound.

Example 25

[2R-(2α,6α, 7β,)]3-[(Acetyloxy)methyl]-8-oxo-7-[[4-[(2,2,2-trichloroethoxy)carbonyl]-2-thiazolyl]amino]-5-thia-1-azabicyclo-[4.2.0]oct-3-ene-2-carboxylic acid, diphenylmethyl ester

A mixture of 1.33 g of (6R-trans)-3-[(acetyloxy)methyl]-7-[(aminothioxomethyl)amino]-8-oxo-5-thia-1-azabicyclo[4.2.0]oct-2-ene-2-carboxylic acid, diphenylmethyl ester, 850 mg of 3-bromo-2-oxopropanoic acid, 2,2,2-trichloroethyl ester, 320 mg of potassium carbonate and 20 ml of acetonitrile was reacted as described in Example 18,giving 350 mg of the desired compound.

Example 26

(6R-trans)-3-[(Acetyloxy)methyl]-8-oxo-7-[[4-[(2,2,2-trichloroethoxy)carbonyl]-2-thiazolyl]amino]-5-thia-1-azabicyclo-[4.2.0]oct-2-ene-2-carboxylic acid

A mixture of 307 mg of (2R-2α,6α,7β)]-3-[(acetyloxy)methyl]-8-oxo-7-[[4-[(2,2,2-trichloroethoxy)carbonyl]-2-thiazolyl]amino]-5-thia-1-azabicyclo[4.2.0]oct-2-ene-2-carboxylic acid, diphenylmethylester, 1 ml of trifluoroacetic acid,0.25 ml of anisole and 1 ml of dichloromethane was reacted as described in Example25, giving 185 mg of the desired product.

Example 27

(6R-trans)-3-[(Acetyloxy)methyl]-8-oxo-7-[[4-[[(phenylmethyl)amino]carbonyl]-2-thiazolyl]-amino]-5-thia-1-azabicyclo[4.2.0]oct-2-ene-2-carboxylic acid, diphenylmethyl ester

A mixture of 176 mg of (6R-trans)-3-[(acetyloxy)-methyl]-7-[(4-carboxy-2-thiazolyl)amino]-8-oxo-5-thia-1-aza-bicyclo[4.2.0]oct-2-ene-2-carboxylic acid, diphenylmethyl

ester, 33.3 mg of benzylamine, 79 mg of 2-ethoxy-1(2H)-quinolinecarboxylic acid, ethyl ester and 3 ml of dichloromethane was stirred at room temperature for 1 hour, then added to water and extracted with dichloromethane. The dichloromethane extract was evaporated, giving the desired compound.

## Example 28

### (6R-trans)-3-[(Acetyloxy)methyl]-8-oxo-7-[[4-[[(phenylmethyl)amino]carbonyl]-2-thiazolyl]-amino]-5-thia-1-azabicyclo[4.2.0]oct-2-ene-2-carboxylic acid

A mixture of 175 mg of (6R-trans)-3-[(acetyloxy)-methyl]-8-oxo-7-[[4-[[(phenylmethyl)amino]carbonyl]-2-thiazolyl]amino]-5-thia-1-azabicyclo[4.2.0]oct-2-ene-2-carboxylic acid, diphenylmethyl ester, trifluoroacetic acid and anisole in dichloromethane was stirred for 45 minutes. The addition of dichloromethane, ether and petroleum ether caused precipitation. The solid was collected and washed with ether and petroleum ether, giving 105 mg of the desired product.

## Example 29

### (6R-trans)-3-[(Acetyloxy)methyl]-7-[(4-methyl-2-thiazolyl)amino]-8-oxo-5-thia-1-azabicyclo-[4.2.0]oct-2-ene-2-carboxylic acid

A mixture of 210 mg of (6R-trans)-3-[(acetyloxy)-methyl]-7-[(4-methyl-2-thiazolyl)amino]-8-oxo-5-thia-1-azabicyclo[4.2.0]oct-2-ene-2-carboxylic acid, diphenylmethyl ester, trifluoroacetic acid and anisole in dichloromethane was reacted as described in Example 28, giving 123 mg of the desired product.

Example 30

(6R-trans)-3-[(Acetyloxy)methyl]-7-[[[[(dimethylamino)methylene]amino]thioxomethyl]amino]-8-oxo-5-thia-1-azabicyclo[4.2.0]oct-2-ene-2-carboxylic acid, diphenylmethyl ester

A 650 mg portion of N,N-dimethylformamide diethylacetal in 10 ml of dichloromethane was added dropwise to a solution of 2 g of (6R-trans)-3-[(acetyloxy)-methyl-7-[(aminothioxomethyl)amino]-8-oxo-5-thia-1-azabicyclo[4.2.0]oct-2-ene-2-carboxylic acid, diphenylmethyl ester in 35 ml of dichloromethane. The mixture was stirred for 20 minutes, then filtered, evaporated and the residue purified by flash chromatography using the system ethyl acetate:petroleum ether (1:1) and giving 1.08 g of the desired product.

Example 31

[2R-(2α,6α, 7β)]-3-[(Acetyloxy)methyl]-7-[(5-benzolyl-2-thiazolyl)amino]-8-oxo-5-thia-1-azabicyclo[4.2.0]oct-3-ene-2-carboxylic acid, diphenylmethyl ester

A mixture of 940 mg of (6R-trans)-3-[(acetyloxy)methyl]-7-[[[[(dimethylamino)methylene]amino]thioxomethyl]-amino]-8-oxo-5-thia-1-azabicyclo[4.2.0]oct-2-ene-2-carboxylic acid, diphenylmethyl ester, 380 mg of 2-bromo-1-phenylethanone, 235 mg of potassium carbonate and 30 ml of acetonitrile was reacted as described in Example 30, giving 900 mg of the desired compound.

Example 32

(6R-trans)-3-[(Acetyloxy)methyl]-7-[(5-benzoyl-2-thiazolyl)amino]-8-oxo-5-thia-1-azabicyclo-[4.2.0]oct-2-ene-2-carboxylic acid, diphenylmethyl ester

A mixture of 720 mg of (6R-trans)-3-[(acetyloxy)-methyl]-7-[[[[(dimethylamino)methylene]amino]thioxomethyl]-amino]-8-oxo-5-thia-1-azabicyclo[4.2.0]oct-2-ene-2-carboxylic acid, diphenylmethyl ester, 260 mg of 2-bromo-1-phenylethanone and 90 mg of potassium carbonate in 15 ml of dry acetonitrile was stirred for about 1 hour, then filtered and the filtrate evaporated, giving 526 mg of the desired product.

Example 33

(6-R-trans)-3-[(Acetyloxy)methyl]-7-[(5-acetyl-2-thiazolyl)amino]-8-oxo-5-thia-1-azabicyclo-[4.2.0]oct-2-ene-2-carboxylic acid, diphenylmethyl ester

A mixture of 585 mg of (6R-trans)-3-[(acetyloxy)-methyl]-7-[[[[(dimethylamino)methylene]amino]thioxomethyl]-amino]-8-oxo-5-thia-1-azabicyclo[4.2.0]oct-2-ene-2-carboxylic acid, diphenylmethyl ester, 127 mg of propionyl chloride, 188 mg of sodium iodide and 15 ml of acetontrile was stirred for 90 minutes, then 99 mg of pyridine was added. This mixture was stirred for 90 minutes, then dichloromethane was added and the mixture was refluxed for 20 minutes. The solution was washed with 10% hydrochloric acid, brine and aqueous sodium bicarbonate, dried and evaporated, giving 418 mg of the desired product.

Example 34

(6R-trans)-3-[(Acetyloxy)methyl]-7-[(5-acetyl-2-thiazolyl)amino]-8-oxo-5-thia-1-azabicyclo-[4.2.0]oct-2-ene-2-carboxylic acid

A mixture of 255 mg of (6R-trans)-3-[(acetyloxy)-methyl]-7-[(5-acetyl-2-thiazolyl)amino]-8-oxo-5-thia-1-azabicyclo[4.2.0]oct-2-ene-2-carboxylic acid, diphenylmethyl ester was reacted with anisole and trifluoroacetic acid in dichloromethane with stirring at $0^{o}C$ for 30 minutes, then at room temperature for 30 minutes. The addition of ether and petroleum ether gave a precipitate which was washed with ether, giving 135 mg of the desired product as a pale yellow powder.

Example 35

(6R-trans)-3-[(Acetyloxy)methyl]-7-[(5-benzoyl-2-thiazolyl)amino]-8-oxo-5-thia-1-azabicyclo-[4.2.0]oct-2-ene-2-carboxylic acid

A 235 mg portion of (6R-trans)-3-[(Acetyloxy)-methyl]7-[(5-benzoyl-2-thiazolyl)amino]-8-oxo-5-thia-1-azabicyclo[4.2.0]oct-2-ene-2-carboxylic acid, diphenylmethyl ester was reacted with anisole and trifluoroacetic acid in dichloromethane as described in Example 34, giving 143 mg of the desired product as a pale yellow powder.

Example 36

(6R-trans)-3-[(Acetyloxy)methyl]-8-oxo-7-(1,2,4-thiadiazol-5-ylamino)-5-thia-1-azabicyclo-[4.2.0]oct-2-ene-2-carboxylic acid, diphenylmethyl ester

A mixture of 60 mg of (6R-trans)-3-[(acetyloxy)-methyl]-7-[[[[(dimethylamino)methylene]amino]thioxomethyl]-amino]-8-oxo-5-thia-1-azabicyclo[4.2.0]oct-2-ene-2-carboxylic acid, diphenylmethyl ester, 14 mg of hydroxylamine-O-sulfonic acid and 17.14 mg of pyridine in a mixture of 3 ml of ethanol and 1 ml of methanol was stirred at room temperature. A 0.5 ml portion of dioxane was added to enhance solubility. After 1 hour the mixture was concentrated, dichloromethane added to the residue, the solution washed with 10% hydrochloric acid and brine, then decolorized, filtered and evaporated, giving the desired product.

WHAT IS CLAIMED IS:

1. A compound of the following formulae:

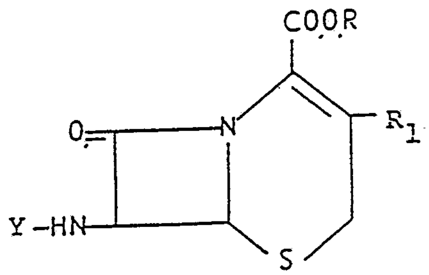

, or

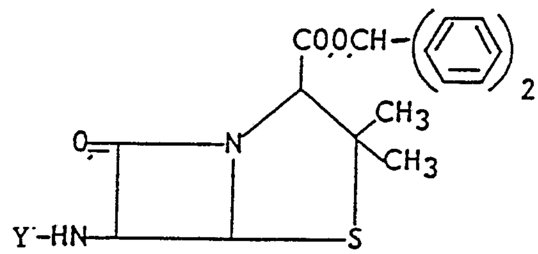

wherein R is hydrogen or diphenylmethyl; $R_1$ is hydrogen, alkyl($C_1$-$C_3$), vinyl, acetyloxymethyl, diphenylmethyl, $CH_2OCH_2CH_2Si(CH)_3$, $Si(CH_3)_3$ or

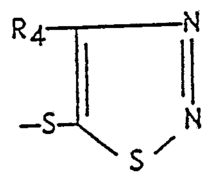

,

wherein $R_4$ is hydrogen or alkyl ($C_1$-$C_6$); and Y is

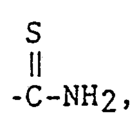

,

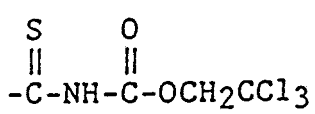

;

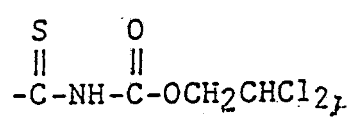

,

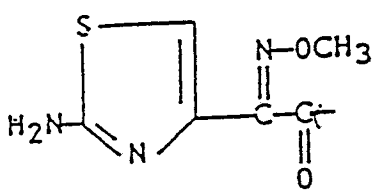

or

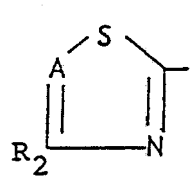

wherein A is HC, N or $R_3$-C, wherein $R_3$ is acetyl or benzyl; and $R_2$ is alkyl ($C_1$-$C_3$), phenyl, carboxylic acid, (2,2,2-trichloroethoxy)carbonyl, [2-(trimethylsilyl)ethoxy]carbonyl, phenylmethylamino carbonyl or ethoxycarbonyl.

2. A compound according to claim 1, 4-chloro-2-(Z)-hydroxyimino-3-oxobutanoate, 4-chloro-2-(Z)-methoxyimino-3-oxobutanoate, 4-chloro-2-(Z)-methoxyimino-3-oxobutanoic acid, 7-[4-chloro-2-(Z)-methoxyimino-3-oxobutyramido]-3[(1,2,3-thiadiazol-5-yl)thiomethyl]ceph-3-em-4-carboxylic acid, diphenylmethyl 7-[4-chloro-2-(Z)-methoxyimino-3-oxobutyramido]-3[1,2,3-thiadiazol-5-yl)thiomethyl]ceph-3-em-4-carboxylate, 2-(trimethylsilyl)ethoxymethyl 7-[4-chloro-2-(Z)-methoxyimino-3-oxobutyramido]-3-[(1,2,3-thiadiazol-5-yl)thiomethyl}ceph-3-em-4-carboxylate or 2-(trimethylsilyl)ethoxymethyl 7-[2-(2-aminothiazol-4-yl)-(Z)-2-methoxyiminoacetamide]-3-[1,2,3-thiadiazol-5-yl)thiomethyl]ceph-3-em-4-carboxylate.

3. The compound according to claim 1, (6R-trans)-3-[(acetyloxy)methyl]-8-oxo-7-[[thioxo[[2,2,2-trichloroethoxy)carbonyl]amino]methyl]amino]-5-thia-1-azabicyclo[4.2.0]oct-2-ene-2-carboxylic acid, diphenylmethyl ester, (6R-trans)-3-[(acetyloxy)methyl]-7-[(aminothioxomethyl)amino]-8-oxo-5-thia-1-azabicyclo-[4.2.0]oct-2-ene-2-carboxylic acid, diphenylmethyl ester, (6R-trans)-3-[(acetyloxy)methyl]-7-[[[[(2,2-dichloroethoxy)carbonyl]amino]thioxomethyl]amino]-8-oxo-5-thia-1-azabicyclo[4.2.0]oct-2-ene-2-carboxylic acid, diphenylmethyl ester.

4. The compound according to claim 1, [2S-(2α,5α,6β)]-3,3-dimethyl-7-oxo-6-[[thioxo[[2,2,2-trichloroethoxy)carbonyl]amino]methyl]amino]-4-thia-1-azabicyclo[3.2.0]heptane-2-carboxylic acid, diphenylmethyl ester or 6-[(aminothioxomethyl)amino]-3,3-dimethyl-7-oxo-4-thia-1-azabicyclo[3.2.0]heptane-2-carboxylic acid, diphenylmethyl ester.

5. The compound according to claim 1, (6R-trans)-3-[(acetyloxy)methyl]-8-oxo-7-[(4-phenyl-2-thiazolyl)amino]-5-thia-1-azabicyclo[4.2.0]oct-2-ene-2-carboxylic acid, diphenylmethyl ester, (6R-trans)-3-[(acetyloxy)methyl]-7-[(4-methyl-2-thiazolyl)amino]-8-oxo-5-thia-1-azabicyclo[4.2.0]oct-2-ene-2-carboxylic acid, diphenylmethyl ester, (6R-trans)-3-[(acetyloxy)-methyl]-7-[(4-methyl-2-thiazolyl)amino]-8-oxo-5-thia-1-azabicyclo[4.2.0]oct-2-ene-2-carboxylic acid, diphenylmethyl 5-oxide, (6R-trans)-3-[(acetyloxy)methyl]-8-oxo-7-[[(4-[[2-trimethylsilyl)ethoxy]carbonyl)-2-thiazolyl]amino]-5-thia-1-azabicyclo[4.2.0]oct-2-ene-2-carboxylic acid, diphenylmethyl ester, (6R-trans)-3-[(acetyloxy)methyl]-7-[[(4-carboxy-2-thiazolyl)amino]-8-oxo-5-thia-1-azabicyclo[4.2.0]oct-2-ene-2-carboxylic acid, diphenylmethyl ester, [2R-(2α,6α,7β)]-3-[(acetyloxy)methyl]-8-oxo-7-[[2,2,2-trichloroethoxy)carbonyl]-2-thiazolyl]amino]-5-thia-1-azabicyclo[4.2.0]oct-2-ene-2-carboxylic acid, diphenylmethyl ester, (6R-trans)-3-[(acetyloxy)methyl]-8-oxo-7-[[4-[[(phenylmethyl)amino]-carbonyl]-2-thiazolyl]amino]-5-thia-1-azabicyclo[4.2.0]-oct-2-ene-2-carboxylic acid, diphenylmethyl ester, (6R-trans)-3-[(acetyloxy)methyl]-8-oxo-7-[[4-[[(phenylmethyl)amino]carbonyl-2-thiazolyl]amino]-5-thia-1-azabi-cyclo[4.2.0]oct-2-ene-2-carboxylic acid or (6R-trans)-3-[(acetyloxy)methyl]-7-[(4-methyl-2-thiazolyl)amino]-8-oxo-5-thia-1-azabicyclo[4.2.0]oct-2-ene-2-carboxylic acid.

6. The compound according to claim 1, (6R-trans)-3-[(acetyloxy)methyl]-7-[(4-ethoxycarbonyl)-2-thiazolyl)amino]-8-oxo-5-thia-1-azabicyclo[4.2.0]oct-2-ene-2-carboxylic acid, (6R-trans)-3-[(acetyloxy)methyl]-8-oxo-7-[(4-phenyl-2-thiazolyl)amino]-5-thia-1-azabi-cyclo[4.2.0]oct-2-ene-2-carboxylic acid, (6R-trans)-3-[(acetyloxy)methyl]-7-[(4-carboxy-2-thiazolyl]amino]-8-oxo-5-thia-1-azabicyclo[4.2.0]oct-2-ene-2-carboxylic acid or (6R-trans)-3-[(acetyloxy)methyl]-8-oxo-7-[[(4-[2,2,2-trichloroethoxy)carbonyl]-2-thiazolyl]amino]-5-thia-1-azabicyclo[4.2.0]oct-2-ene-2-carboxylic acid.

7. The compound according to claim 1, [2R-(2α,6α,7β)]-3-[(acetyloxy)methyl]-7-[(5-benzoyl-2-thiazolyl)amino]-8-oxo-5-thia-1-azabicyclo[4.2.0]oct-3-ene-2-carboxylic acid, diphenylmethyl ester, (6R-trans)-3-[(acetyloxy)methyl]-7-[(5-benzoyl-2-thiazolyl)amino]-8-oxo-5-thia-1-azabicyclo[4.2.0]oct-2-ene-2-carboxylic acid, diphenylmethyl ester, (6R-trans)-3-[(acetyloxy)-methyl]-7-[(5-acetyl-2-thiazolyl)amino]-8-oxo-5-thia-1-azabicyclo[4.2.0]oct-2-ene-2-carboxylic acid, diphenylmethyl ester, (6R-trans)-3-[(acetyloxy)methyl]-7-[(5-acetyl-2-thiazolyl)amino]-8-oxo-5-thia-1-azabicyclo-[4.2.0]oct-2-ene-2-carboxylic acid, (6R-trans)-3-[(acetyloxy)methyl]-7-[(5-benzoyl-2-thiazolyl)amino]-8-oxo-5-thia-1-azabicyclo[4.2.0]oct-2-ene-2-carboxylic acid, (6R-trans)-3-[(acetyloxy)methyl]-8-oxo-7-(1,2,4-thiadiazol-5-ylamino)-5-thia-1-azabicyclo[4.2.0]oct-2-ene-2-carboxylic acid, diphenylmethyl ester or (6R-trans)-3-[(acetyloxy)methyl]-7-[[[[(dimethylamino)-methylene]amino]thioxomethyl]amino]-8-oxo-5-thia-1-azabicyclo[4.2.0]oct-2-ene-2-carboxylic acid, diphenylmethyl ester.

8. A process for producing a compound of the formula:

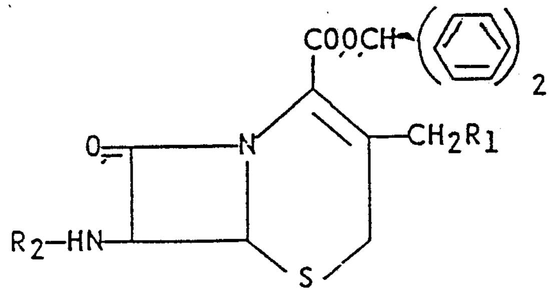

wherein $R_1$ is hydrogen, alkyl($C_1$-$C_3$), vinyl, acetyloxy or

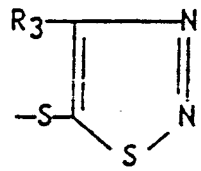

,

where $R_3$ is selected from hydrogen or alkyl($C_1$-$C_6$); and

S S O S O

$R_2$ is $-C-NH_2$, $-C-NH-C-OCH_2CCl_3$ or $-C-NH-C-OCH_2CHCl_2$, which comprises reacting a 7-aminocephalosporanic acid, diphenylmethyl ester where $R_1$ is as described above with carbon (isothiocyanatidic) acid, 2,2,2-trichloroethyl ester in dichloromethane, giving a compound of the above formula where $R_1$ is as described above and $R_2$ is

S O

$-C-NH-C-OCH_2CCl_3$, further reacting this compound with zinc dust, glacial acetic acid and 1M potassium dihydrogen phosphate in tetrahydrofuran giving products of the above formula where $R_1$ is as described and $R_2$ is

S S O

$-C-NH_2$ or $-C-NH-C-OCH_2CHCl_2$.

9. A process for producing 6-[(aminothioxomethyl)amino]-3,3-dimethyl-7-oxo-4-thia-1-azabicyclo-[3.2.0]heptane-2-carboxylic acid, diphenylmethyl ester which comprises reacting 6-amino-3,3-dimethyl-7-oxo-4-thia-1-azabicyclo[3.2.0]heptane-2-carboxylic acid, diphenylmethyl ester with thiophosgene and ammonium acetate in a mixture of ethyl acetate and water, followed by the incremental addition of ammonium hydroxide and extraction of the product from the organic layer.

10. A process for producing a compound of the formula:

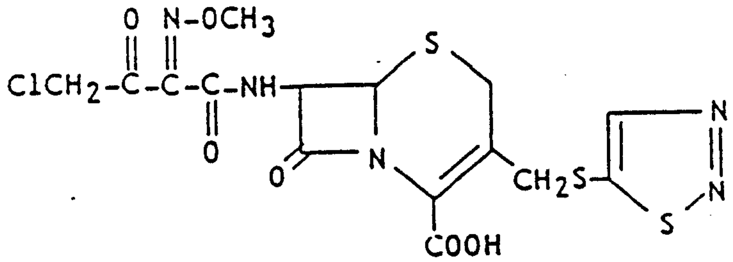

which comprises reaction a (Z)-methoximino acid of the

O N-$OCH_3$

formula $ClCH_2C$-C-COOH with a 7-aminocephalosporin of the formula:

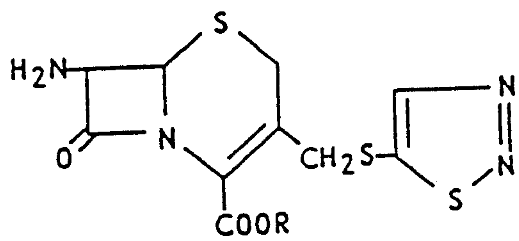

where R is trimethylsilylethoxymethyl ester or benhydryl ester and 1-ethoxycarbonyl-2-ethoxy-1,2-dihydroquinoline giving protected derivatives of the formula:

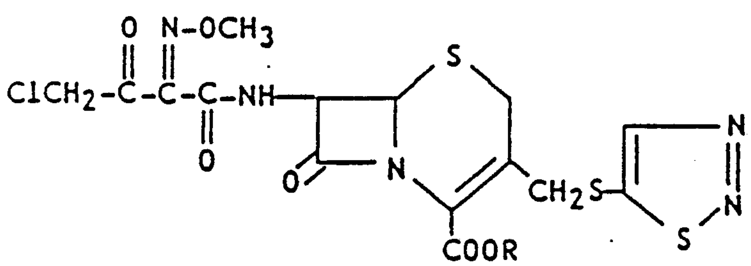

which is then reacted with trifluoroacetic acid to give the desired compound.

11. A process for producing a compound of the formula:

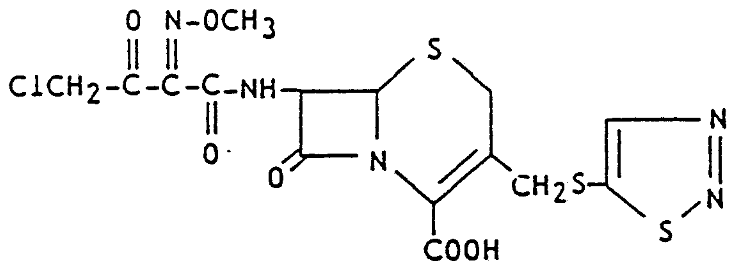

which comprises reacting a compound of the formula $ClCH_2\overset{O}{\overset{\|}{C}}-\overset{N-OCH_3}{\overset{\|}{C}}-COCl$ with a 7-aminocephalosporin of the formula:

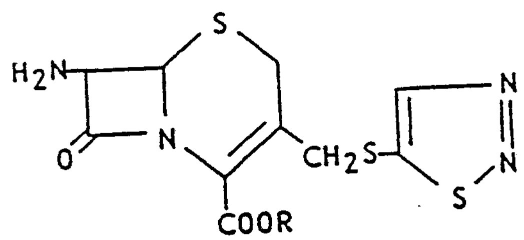

where R is trimethylsilyl and then with trifluoroacetic acid giving the desired compound.

12. A process for producing a compound of the formula:

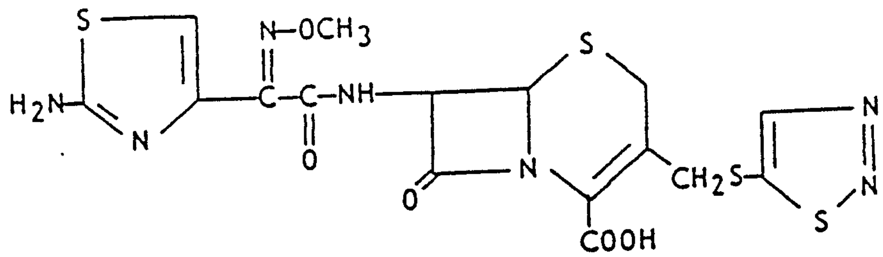

which comprises reacting 2-(trimethylsilyl)ethoxymethyl 7β-[4-chloro-2-(Z)-methoxyimino-3-oxobutyramido]-3-[(1,2,3-thiadiazol-5-yl)thiomethyl]ceph-3-em-4-carboxylate with thiourea giving 2-(trimethylsilyl)ethoxymethyl 7β-[2-(2-aminothiazol-4-yl)-(Z)-2-methoxyiminoacetamido]-3-[(1,2,3-thiadiazol-5-yl)thiomethyl]ceph-3-em-4-carboxylate followed by reaction with trifluoroacetic acid to produce the desired product.

13. A process for producing a compound of the formula:

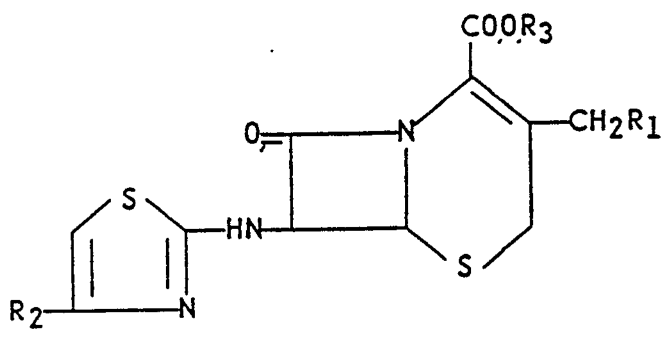

wherein $R_1$ is hydrogen, alkyl($C_1$-$C_3$), vinyl, acetyloxy or

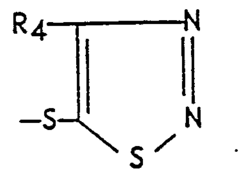

where $R_4$ is hydrogen or alkyl($C_1$-$C_6$); $R_2$ is alkyl($C_1$-$C_3$), phenyl, carboxylic acid, (2,2,2-trichloroethoxy)carbonyl, [2-(trimethylsilyl)ethoxy]carbonyl, phenylmethylamino carbonyl or ethoxycarbonyl; and $R_3$ is hydrogen, an alkali metal or an alkaline earth metal; which comprises reacting a 3-substituted-8-oxo-7-substituted-thioxomethylamino-5-thia-1-azabicyclo-[4.2.0]oct-2-ene-2-carboxylic acid, diphenylmethyl ester of the formula

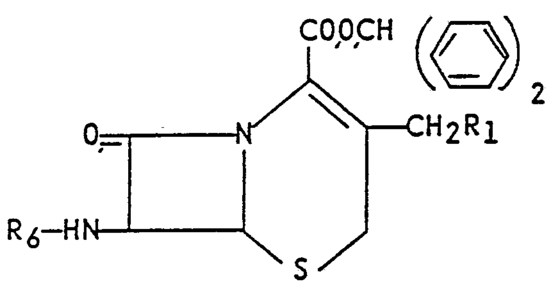

, where $R_1$ is as described above and $R_6$ is $-\overset{S}{\overset{\|}{C}}-NH_2$, $-\overset{S}{\overset{\|}{C}}-NH-\overset{O}{\overset{\|}{C}}-OCH_2CCl_3$ or $-\overset{S}{\overset{\|}{C}}-NH-\overset{O}{\overset{\|}{C}}-OCH_2CHCl_2$ with an $\alpha$-halogenocarbonyl derivative of the formula $R_2COCH_2Br$, where $R_2$ is as described above, and potassium carbonate in acetonitrile, giving a compound of the formula

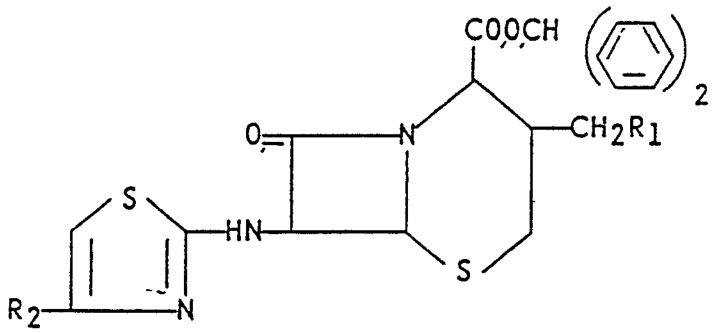

where $R_1$ and $R_2$ are as desctibed above, followed by reaction with trifluoroacetic acid and anisole in dichloromethane at -5 to 45°C, giving the desired antibacterial agents where $R_3$ is hydrogen, and dissolving said resulting antibacterial agent in water, adding an alkali metal bicarbonate, stirring and then evaporating the water to produce the desired antibacterial agents where $R_3$ is alkali metal or alkaline earth metal.

14. A process for producing a compound of the formula:

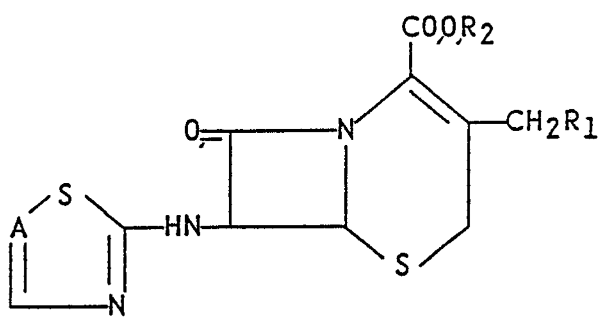

wherein $R_1$ is hydrogen, alkyl($C_1$-$C_3$), vinyl, acetyloxy or

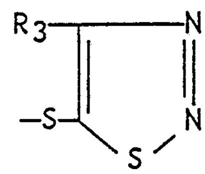
,

where $R_3$ is hydrogen or alkyl($C_1$-$C_6$); $R_2$ is hydrogen or diphenylmethyl; and A is N, or $R_4$-C, where $R_4$ is acetyl or benzoyl; which comprises reacting a 3-substituted-7-[(aminothioxomethyl)amino]-8-oxo-5-thia-1-azabicyclo-[4.2.0]oct-2-ene-2-carboxylic acid, diphenylmethyl ester of the formula

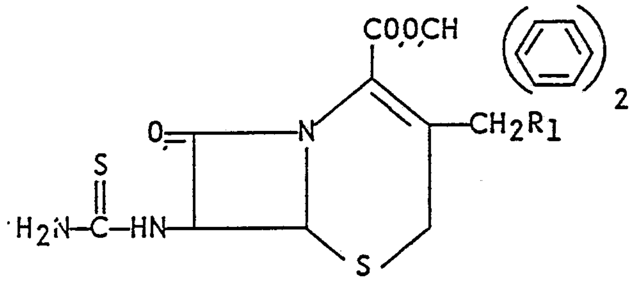
, where $R_1$ is as

described above, with N,N-dimethylformamide diethylacetal giving the corresponding 3-substituted-7-[[[[(di-methylamino)methylene]amino]thioxomethyl]amino]-8-oxo-5-thia-1-azabicyclo[4.2.0]oct-2-ene-2-carboxylic acid, diphenylmethyl ester of the formula

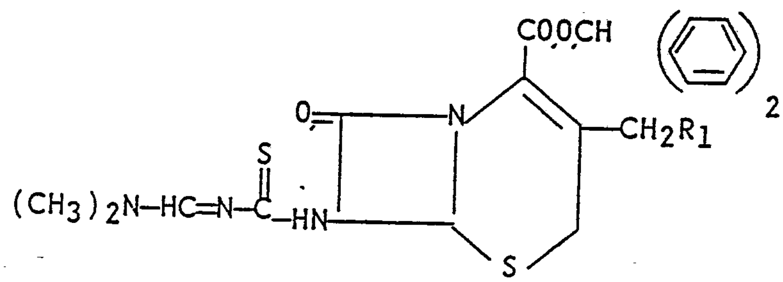

which is then reacted with a 2-bromo-substituted ethanone of the formula $R_4COCH_2Br$, where $R_4$ is as described above, and potassium carbonate in acetonitrile giving the desired products.